# EUROPEAN PATENT APPLICATION

(11) **EP 4 825 846 A1**
(43) Date of publication of application: **21.10.2026**
(21) Application number: 24902742.6
(22) Date of filing: 09.12.2024
(51) Int. Cl.: C07D 239/54, C07C 271/28, C07C 271/32, C07C 271/40, A01N 35/08, A01P 3/00

(54) **PREPARATION METHOD FOR URACIL COMPOUND AND USE OF URACIL COMPOUND**

(30) Priority: 12.12.2023 CN 202311700459
(71) Applicant: Jiangsu Flag Chemical Industry Co., Ltd, Nanjing, Jiangsu 210000 (CN); Anhui Neotec Co., Ltd., Huaibei, Anhui 235000 (CN)
(72) Inventor: ZHANG, Pu, Nanjing, Jiangsu 210000 (CN); ZHANG, Jian, Nanjing, Jiangsu 210000 (CN); JIN, Yucun, Nanjing, Jiangsu 210000 (CN); YAO, Kaicheng, Nanjing, Jiangsu 210000 (CN); LI, Bin, Nanjing, Jiangsu 210000 (CN); WU, Yaojun, Nanjing, Jiangsu 210000 (CN)
(74) Representative: Zimmermann & Partner Patentanwälte mbB
(86) International application number: PCT/CN2024/137684
(87) International publication number: WO 2025/124321

(57) **Abstract**

The present invention relates to the field of pesticides, and in particular, to a method for preparing a compound of formula **(III).** The compound of formula **(III)** can be used to prepare novel and efficient uracil herbicides. The present invention further provides an intermediate compound used in the method, preparation method thereof and fungicidal use thereof. The synthesis route is simple and the application prospect is broad.

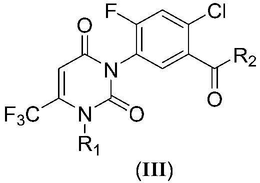

## Description

### TECHNICAL FIELD

The present invention relates to the field of pesticides, and in particular, to a method for preparing a compound of formula (**III**). The compound of formula (**III**) can be used to prepare novel and efficient uracil herbicides. The present invention further provides an intermediate compound used in the method, preparation method thereof and fungicidal use thereof. The synthesis route is simple and the application prospect is broad.

### BACKGROUND

WO2022166938A1 specifically discloses a method for preparing a uracil compound containing carboxylate fragment using ethyl 2-chloro-4-fluoro-5-aminobenzoate as a raw material. This method requires many times of protections and deprotections of carboxylic acid, which increases reaction steps, resulting in a 9-step reaction from the preparation of ethyl 2-chloro-4-fluoro-5-aminobenzoate to a final product. The total yield of the reaction is low, and column chromatography purification is required, which is not suitable for industrial production (see compound 207 in Example 32):

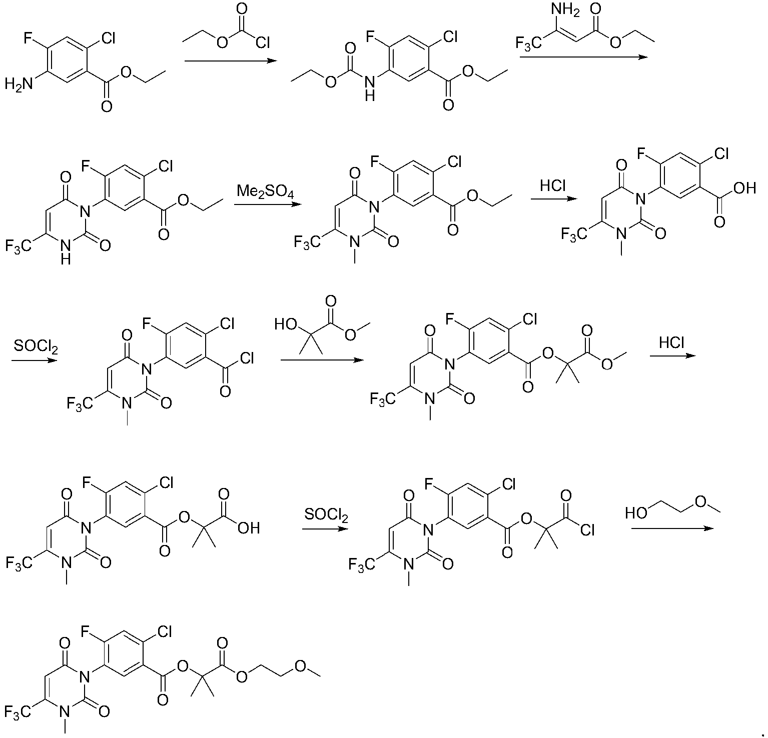

WO2001083459A2 specifically discloses a method for preparing herbicide Saflufenacil using 2-chloro-4-fluoro-5-aminobenzoic acid as raw material. This method requires the use of raw material 2-(dimethylamino)-4-(trifluoromethyl)-6H-1,3-oxazin-6-one which is not easy to prepare, as well as expensive and environmentally unfriendly BBr₃, and the reaction between each step cannot be carried out continuously, requiring additional post-treatment, which is not suitable for industrial production:

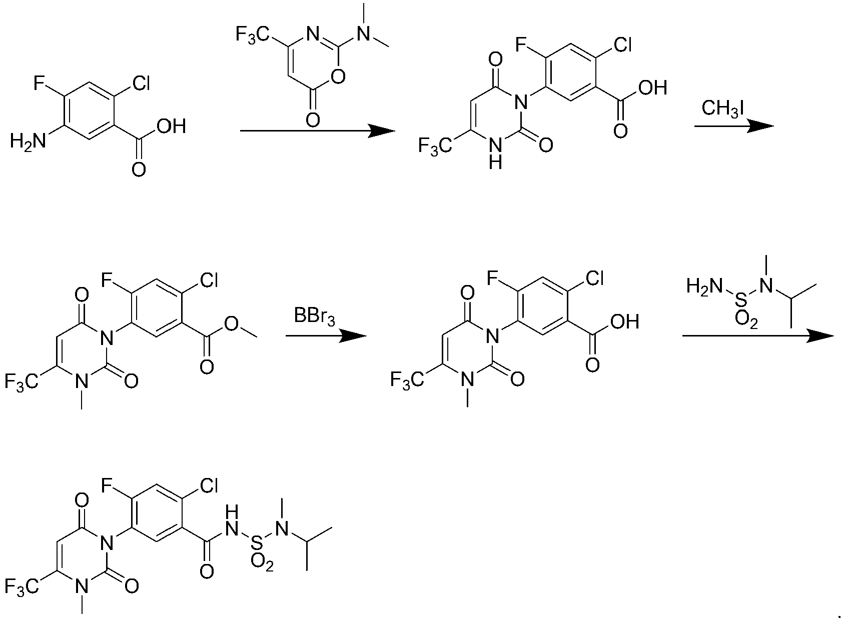

WO2022201155A1 specifically disclosed a method for preparing herbicide Saflufenacil using ethyl 2-chloro-4-fluoro-5-aminobenzoate as a raw material. This method requires the protection and deprotection of the carboxyl group, which increases reaction steps and has poor atomic economy. When ethyl 2-chloro-4-fluoro-5-aminobenzoate reacts with ethyl chloroformate, a large amount of base (such as N,N-diethylaniline) is added as an acid binding agent, which increases the generation of by-products and increases the difficulty and cost of treatment. Moreover, when 2-chloro-5-(2,6-dioxo-4-(trifluoromethyl)-3,6-dihydropyrimidin-1(2H)-yl)-4-fluorobenzoic acid reacts with NH₂SO₂N(CH₃)*i*-Pr, a condensing agent (such as 1,1'-carbonyldiimidazole (CDI)) is added which is expensive and difficult to remove, and the yield of this step is only 70%:

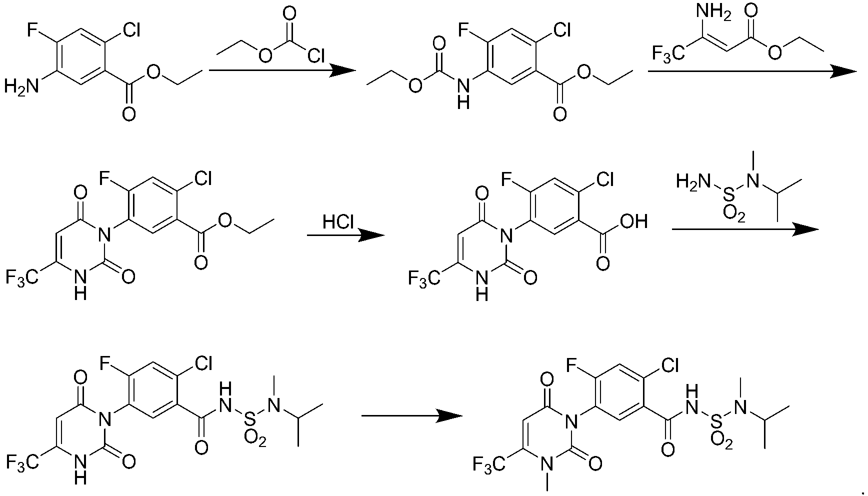

The existing methods for preparing uracil compound herbicides require esterification protection of benzoic acid before synthesizing a uracil ring or N-methylation of a uracil ring, followed by one-step hydrolysis reaction of benzoate, and finally coupling with a corresponding fragment to synthesize the final compound.

There are problems such as poor atom economy benefits, long steps, high cost, low yield, environmental concerns, difficult post-treatment, and discontinuous reaction. At present, the key step in the preparation of a uracil compound herbicide is the synthesis of the uracil ring. Most of the reported methods for synthesizing the ring of the uracil compound herbicide are relatively single, which is not conducive to industrial development.

### SUMMARY

The technical problem to be solved by the present invention is to provide a method for preparing a uracil compound and use of intermediates for preparing fungicides and uracil herbicides, in response to the drawbacks of the prior art.

The technical solution of the present invention to solve the above technical problem is as follows.

The present invention provides a method for preparing a compound of formula (**III**) with the following reaction scheme:

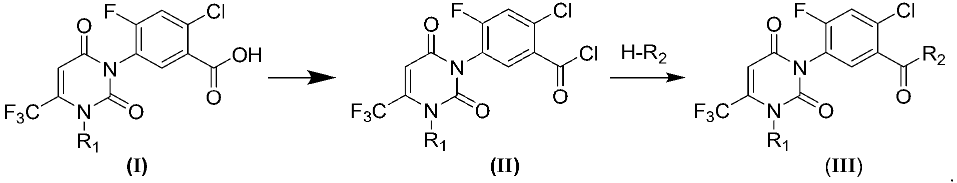

The compound of formula (**I**) reacts with a chlorinating reagent in the presence of a base A in an organic solvent A at 20°C-120°C to give the compound of formula (**II**); wherein, the organic solvent A is selected from at least one of dichloromethane, chloroform, 1,2-dichloroethane, chlorobenzene, dichlorobenzene, toluene or xylene; the base A is selected from at least one of N,N-dimethylformamide (DMF), 4-pyrrolidinopyridine (4-PPY) or 4-dimethylaminopyridine (DMAP); the molar amount of base A is 0.001-0.2 equivalent of the compound of formula (**I**); the chlorinating reagent is selected from SOCl₂, POCl₃, PCl₅, ClCOCOCl, COCl₂, ClCOOCCl₃ or Cl₃COCOOCCl₃.

Base A, as a catalyst in the preparation of the compound of formula (**II**)**,** can reduce reaction time, lower reaction temperature, and improve the conversion rate during the preparation of the compound of formula (**II**). And in the continuous reaction, base A can continue to be used as a catalyst in the preparation of the compound of formula (**III**), reducing the hydrolysis of the compound of formula (**II**) and increasing the conversion rate and yield during the preparation of the compound of formula (**III**).

The compound of formula (**II**) reacts with H-R₂ in the presence of a base B in an organic solvent B at 0°C-120°C to give the compound of formula (**III**); wherein, the organic solvent B is selected from at least one of dichloromethane, chloroform, 1,2-dichloroethane, chlorobenzene, dichlorobenzene, toluene, xylene, tetrahydrofuran or 2-methyltetrahydrofuran; the base B is selected from at least one of 4-dimethylaminopyridine, trimethylamine, triethylamine, diisopropylethylamine, pyridine, 2-methylpyridine, 3-methylpyridine, 1,8-diazabicyclo[5.4.0]undec-7-ene(DBU) or 2,6-lutidine; the amount of base B is 1-3.0 equivalents of the compound of formula (**II**);
R₁ is selected from H or -CH₃;
R₂ is selected from -OC(CH₃)₂COOCH₃, -OC(CH₃)₂COOCH₂CH₃, - OC(CH₃)₂COOCH₂CH₂OCH₃, -OC(CH₃)₂COOCH₂CH=CH₂ or -NHSO₂N(CH₃)*i*-Pr.

Preferably,
when R₁ is selected from H, R₂ is selected from -OC(CH₃)₂COOCH₃, -OC(CH₃)₂COOCH₂CH₃, -OC(CH₃)₂COOCH₂CH₂OCH₃, -OC(CH₃)₂COOCH₂CH=CH₂ or -NHSO₂N(CH₃)*i*-Pr;
when R₁ is selected from -CH₃, R₂ is selected from -OC(CH₃)₂COOCH₃, - OC(CH₃)₂COOCH₂CH₃, -OC(CH₃)₂COOCH₂CH₂OCH₃, -OC(CH₃)₂COOCH₂CH=CH₂ or - NHSO₂N(CH₃)*i*-Pr.

Some compounds (**III**) prepared by the present invention are shown in Table 1.

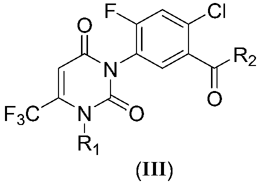

**Table 1 some structures of compound of general formula (III)**

| NO. | R₁ | R₂ |
|---|---|---|
| **III-1** | H | 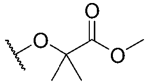 |
| **III-2** | H | 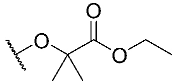 |
| **III-3** | H | 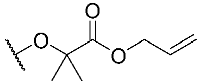 |
| **III-4** | H | 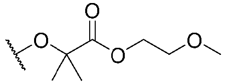 |
| **III-5** | H | 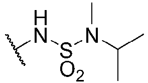 |
| **III-6** | CH₃ | 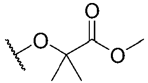 |
| **III-7** | CH₃ | 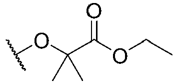 |
| **III-8** | CH₃ | 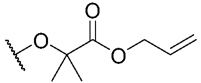 |
| **III-9** | CH₃ | 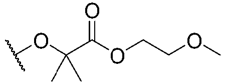 |
| **III-10** | CH₃ | 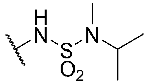 |

In the compound of formula (**III**), when R₁ is selected from H, it can react with the methylation reagent CH₃-X to prepare the compound of formula (**III**) with R1 as -CH₃. The specific operation is carried out according to the method described in WO2001083459A3, and the reaction scheme is as follows:

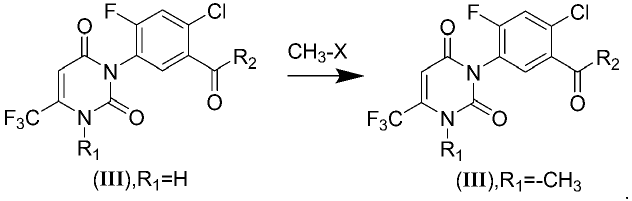

In the above reaction, R₂ in the compound of formula (**III**) is selected from -OC(CH₃)₂COOCH₃, - OC(CH₃)₂COOCH₂CH₃, -OC(CH₃)₂COOCH₂CH₂OCH₃, -OC(CH₃)₂COOCH₂CH=CH₂ or - NHSO₂N(CH₃)*i*-Pr, and X is selected from halogen, -OSO₂OCH₃, -OMs, -OTs or -OTf. Among them: -OMs is -methanesulfonate; -OTs is -tosylate, with the chemical formula of*p*-CH₃PhSO₂O-; -OTf is -trifluoromethanesulfonate, with the chemical formula of CF₃SO₂O-.

Compared with the method described in WO2022166938A1, the above method avoids the protection of carboxyl group, reduces reaction steps, increases the atomic utilization rate, and improves the yield and purity of the product. Compared with the methods described in WO2001083459A2 and WO2022201155A1, the above method avoids the use of expensive and difficult-to-remove condensing agents or environmentally unfriendly reagents. At the same time, when R₁ is selected from -CH₃, it can also avoid the selection of uracil ring N-methylation or - NHSO₂N(CH₃)*i*-Pr side chain N-methylation in the methylation reaction in the preparation of herbicide Saflufenacil, reduce the content of side chain N-methylation impurity, decrease the difficulty of purification, and improve the quality of the product, which is suitable for industrial production.

The second aspect of the present invention provides a compound of formula (**II**): 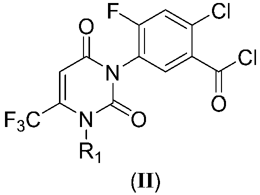 wherein R₁ is selected from H.

The present invention also provides the use of the compound of formula (**II**) with R₁ selected from H in the preparation of the compound of formula (**III**): 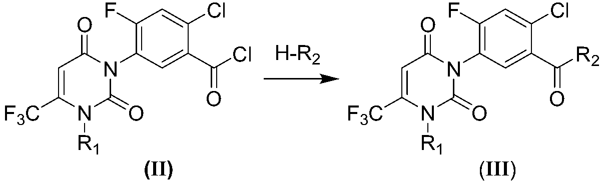 wherein R₁ is selected from H, and R₂ is selected from -OC(CH₃)₂COOCH₃, - OC(CH₃)₂COOCH₂CH₃, -OC(CH₃)₂COOCH₂CH₂OCH₃, -OC(CH₃)₂COOCH₂CH=CH₂ or - NHSO₂N(CH₃)*i*-Pr.

The third aspect of the present invention provides a method for preparing the compound of formula (**I**) with the following reaction scheme:

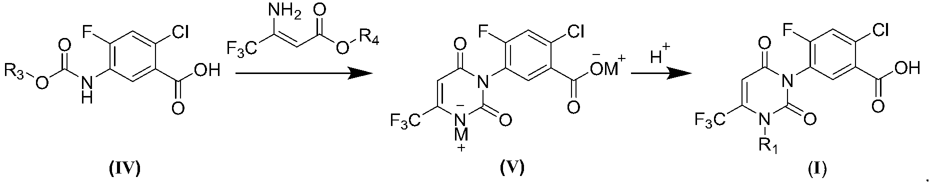

The compound of formula (**IV**) reacts with an R4 ester of 3-amino-4,4,4-trifluorocrotonic acid in the presence of a base C in an organic solvent C at -20°C to the boiling point of the solvent to give a compound of formula (**V**); and the compound of formula (**V**) is treated with an inorganic acid to give the compound of formula (**I**);
wherein,
the base C is selected from at least one of CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the organic solvent C is selected from at least one of methanol, ethanol, isopropanol, tert-butanol, acetonitrile, tetrahydrofuran, 2-methyltetrahydrofuran, 1,4-dioxane, toluene, o-xylene, m-xylene, p-xylene, xylene, 4-methyl-2-pentanone, cyclohexanone, N-methylpyrrolidone, N,N-dimethylformamide, N,N-dimethylacetamide, or dimethyl sulfoxide;
R₁ is selected from H;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl;
R₄ is selected from C₁-C₄ alkyl;
M is selected from K, Na, Li or Cs.

Preferably,
R₃ is selected from C₁-C₇ alkyl, C₃-C₆ cycloalkyl, C₃-C₆ alkenyl, C₃-C₆ alkynyl, phenyl or benzyl;
R₄ is selected from methyl or ethyl;
M is selected from K, or Na;
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the amount of base C is 1.0-3.0 equivalents of the compound of formula (**IV**);
the inorganic acid is selected from hydrochloric acid, sulfuric acid, nitric acid or hydrobromic acid;
the organic solvent C is selected from at least one of methanol, ethanol, tert-butanol, acetonitrile, N-methylpyrrolidone, N,N-dimethylformamide, N,N-dimethylacetamide or dimethyl sulfoxide;
the reaction temperature is selected from 0°C-120°C.

More preferably,
R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl;
R₄ is selected from methyl or ethyl;
M is selected from K, or Na;
the base C is selected from CH₃OK, CH₃CH₂OK, (CH₃)₃COK, CH₃ONa, CH₃CH₂ONa, or (CH₃)₃CONa;
the amount of the base C is 1.0-3.0 equivalents of the compound of formula (**IV**);
the inorganic acid is selected from hydrochloric acid;
the organic solvent C is selected from at least one of methanol, ethanol, acetonitrile, N-methylpyrrolidone, N,N-dimethylformamide, or N,N-dimethylacetamide;
the reaction temperature is selected from 0°C-100°C.

The present invention also provides a method for preparing a compound of formula (**I**) with the following reaction scheme:

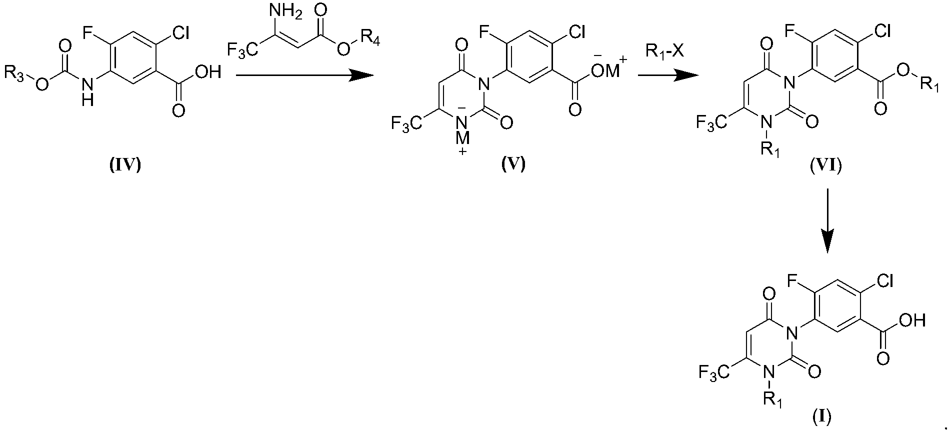

The compound of formula (**IV**) reacts with an R4 ester of 3-amino-4,4,4-trifluorocrotonic acid in the presence of a base C in an organic solvent C at -20°C to the boiling point of the solvent to give a compound of formula (**V**); the compound of formula (**V**) reacts with R₁-X in an organic solvent C at -20°C to the boiling point of the solvent to give the compound of formula (**VI**); and the compound of formula (**VI**) reacts with an inorganic acid in an organic solvent D at 0°C to the boiling point of the solvent to give the compound of formula (**I**);
wherein,
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the organic solvent C is selected from at least one of methanol, ethanol, isopropanol, tert-butanol, acetonitrile, tetrahydrofuran, 2-methyltetrahydrofuran, 1,4-dioxane, toluene, o-xylene, m-xylene, p-xylene, xylene, 4-methyl-2-pentanone, cyclohexanone, N-methylpyrrolidone, N,N-dimethylformamide, N,N-dimethylacetamide, or dimethyl sulfoxide;
the organic solvent D is selected from formic acid or acetic acid;
R₁ is selected from -CH₃;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl;
R₄ is selected from C₁-C₄ alkyl;
M is selected from K, Na, Li or Cs;
X is selected from halogen, -OSO₂OCH₃, -OMs, -OTs or -OTf. Among them: -OMs is - methanesulfonate; -OTs is -tosylate, with the chemical formula of *p*-CH₃PhSO₂O-; -OTf is - trifluoromethanesulfonate, with the chemical formula of CF₃SO₂O-.

Preferably,
R₃ is selected from C₁-C₇ alkyl, C₃-C₆ cycloalkyl, C₃-C₆ alkenyl, C₃-C₆ alkynyl, phenyl or benzyl;
R₄ is selected from methyl or ethyl;
M is selected from K, or Na;
X is selected from Cl, Br, I, -OSO₂OCH₃ or -OMs;
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the amount of the base C is 1.0-3.0 equivalents of the compound of formula (**IV**);
the inorganic acid is selected from hydrochloric acid, sulfuric acid, or nitric acid;
the organic solvent C is selected from at least one of methanol, ethanol, tert-butanol, N-methylpyrrolidone, N,N-dimethylformamide, N,N-dimethylacetamide, or dimethyl sulfoxide;
the organic solvent D is selected from acetic acid;
the reaction temperature is selected from 0-120°C.

More preferably,
R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl;
R₄ is selected from methyl or ethyl;
M is selected from K, or Na;
X is selected from I, or -OSO₂OCH₃;
the base C is selected from CH₃OK, CH₃CH₂OK, (CH₃)₃COK, CH₃ONa, CH₃CH₂ONa, or (CH₃)₃CONa;
the amount of the base C is 1.0-3.0 equivalents of the compound of formula (**IV**);
the inorganic acid is selected from hydrochloric acid, or sulfuric acid;
the organic solvent C is selected from methanol, ethanol, N-methylpyrrolidone, N,N-dimethylformamide, or N,N-dimethylacetamide;
the organic solvent D is selected from acetic acid;
the reaction temperature is selected from 0-110°C.

Compared with the methods described in WO2022166938A1, WO2001083459A2 and WO2022201155A1, the reaction of each step of the above method can be carried out continuously, which reduces the post-treatment, avoids the use of expensive raw materials and environmentally unfriendly reagents, reduces the reaction steps, improves the atomic utilization rate, and increases the reaction yield.

The fourth aspect of the present invention provides a method for preparing a compound of formula (**IV**) with the following reaction scheme:

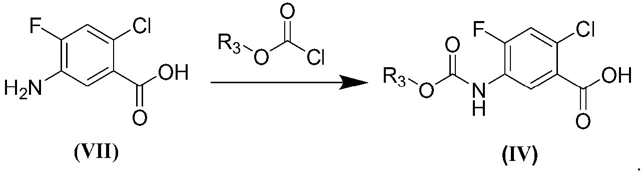

The compound of formula (**VII**) reacts with a chloroformate of formula R3OCOCl in an organic solvent E at -20°C to the boiling point of the solvent to give a compound of formula (**IV**);
wherein,
the organic solvent E is selected from at least one of dichloromethane, chloroform, 1,2-dichloroethane, toluene, xylene, chlorobenzene, dichlorobenzene, N-methylpyrrolidone, ethylene glycol dimethyl ether, ethylene glycol diethyl ether, diethylene glycol diethyl ether, 1,4-dioxane, tetrahydrofuran, or 2-methyltetrahydrofuran;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl.

Preferably,
R₃ is selected from C₁-C₇ alkyl, C₃-C₆ cycloalkyl, C₃-C₆ alkenyl, C₃-C₆ alkynyl, phenyl or benzyl;
the organic solvent E is selected from toluene, ethylene glycol dimethyl ether, 1,4-dioxane, tetrahydrofuran, 2-methyltetrahydrofuran, or N-methylpyrrolidone;
the reaction temperature is selected from 60-110°C.

More preferably,
R₃ is selected from methyl, ethyl, n-propyl, isopropyl, n-butyl, tert-butyl, isobutyl, n-pentyl, n-hexyl, n-heptyl, cyclopentyl, allyl, phenyl or benzyl;
the organic solvent E is selected from ethylene glycol dimethyl ether, 1,4-dioxane, tetrahydrofuran, or 2-methyltetrahydrofuran.

Compared with the methods described in WO2022166938A1 and WO2022201155A1, the above method does not require the use of esterified 2-chloro-4-fluoro-5-aminobenzoic acid (such as ethyl 2-chloro-4-fluoro-5-aminobenzoate) as a raw material. Instead, a selective reaction can be carried out between -NH2 and -COOH of 2-chloro-4-fluoro-5-aminobenzoic acid, avoiding the use of an acid binding agent and the need for esterification protection of benzoic acid, thereby reducing reaction steps, improving atomic utilization rate, increasing product yield and purity, and making it suitable for industrial production. Compared with the method described in WO2001083459A2, the above method avoids the use of raw material 2-(dimethylamino)-4-(trifluoromethyl)-6H-1,3-oxazin-6-one which is expensive and not easy to prepare, and reduces costs.

The fifth aspect of the present invention provides a compound of formula (**IV**): 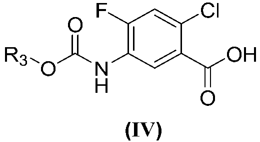 wherein R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl.

Preferably, R₃ is selected from C₁-C₇ alkyl, C₃-C₆ cycloalkyl, C₃-C₆ alkenyl, C₃-C₆ alkynyl, phenyl or benzyl.

More preferably,
R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl.

The present invention also provides use of the compound of formula (**IV**) in the preparation of the compound of formula (**I**).

In the definition of general formula compounds given above, the terms used are gathered and generally defined as follows.

Halogen refers to fluorine, chlorine, bromine, or iodine. Alkyl refers to linear or branched alkyl, such as methyl, ethyl, propyl, isopropyl, n-butyl, tert-butyl or sec-butyl and isomer thereof. Alkenyl refers to linear or branched alkenyl, such as ethenyl, 1-propenyl, 2-propenyl, and different isomers of butenyl, pentenyl, and hexenyl. Alkenyl also includes polyene, such as 1,2-prodienyl and 2,4-hexadienyl. Alkynyl refers to linear or branched alkynyl, such as ethynyl, propynyl, and different isomers of butynyl, pentynyl and hexynyl. Alkynyl also includes polyyne, such as 2,4-hexadiyne. Cycloalkenyl refers to cyclic alkenyl, such as 3-cyclopentenyl. Cycloalkyl refers to substituted or unsubstituted cyclic alkyl, such as cyclobutyl, cyclopentyl, and substituent groups such as methyl, halogen, cyano, etc. Cycloalkyl alkyl refers to substituted or unsubstituted alkyl with a cyclic alkyl, such as cyclopropylmethyl, cyclobutylmethyl, and substituent groups such as methyl, halogen, cyano, etc. Halogenated alkyl refers to linear or branched alkyl on which the hydrogen atoms can be partially or completely replaced by halogen atoms, such as chloropropyl, bromopropyl, etc. Alkoxy-alkyl refers to alkyl-O-alkyl, such as CH₃OCH₂-. Oxygen-containing saturated heterocyclyl refers to a saturated heterocyclic group containing one or more oxygen atoms, such as tetrahydrofuran-2-yl. Oxygen-containing saturated heterocyclyl-alkyl refers to alkyl with oxygen-containing saturated heterocyclyl, such as oxiran-2-ylmethyl.

The above method of the present invention can also include the necessary pretreatment of the above raw materials and the necessary post-treatment of the reaction products. The operation methods of pretreatment and post-treatment include but are not limited to drying, washing, slurrying, filtration, centrifugation, column chromatography, recrystallization, etc. The example part of the present invention provides several specific processing methods, which should not be understood by the person skilled in the art as a limitation to the present invention.

Some compounds of general formula (**IV**) of the present invention can be explained by the specific compounds listed in Table 2, but the present invention is not limited to these compounds.

**Table 2 Structures and ¹H-NMR of some compounds of general formula (IV)**

| NO. | R₃ | ¹H-NMR |
|---|---|---|
| **IV-1** | -CH₃ | ¹H NMR (400 MHz, DMSO-*d6*) δ 13.49 (s, 1H), 9.68 (s, 1H), 8.22 (d, J = 8.5 Hz, 1H), 7.58 (d, J = 10.6 Hz, 1H), 3.69 (s, 3H). |
| **IV-2** | -CH₂CH₃ | ¹H NMR (400 MHz, DMSO-*d6*) δ 13.49 (s, 1H), 9.64 (s, 1H), 8.25 (d, J = 8.5 Hz, 1H), 7.57 (d, J = 10.6 Hz, 1H), 4.16 (q, J = 7.1 Hz, 2H), 1.25 (t, J = 7.1 Hz, 3H). |
| **IV-3** | -CH₂CH₂CH₃ | ¹H NMR (400 MHz, DMSO) δ 13.47 (s, 1H), 9.64 (s, 1H), 8.23 (d, J = 8.4 Hz, 1H), 7.57 (d, J = 10.6 Hz, 1H), 4.06 (t, J = 6.7 Hz, 2H), 1.69 - 1.54 (m, 2H), 0.95 - 0.82 (m, 3H). |
| **IV-4** | -CH(CH₃)₂ | ¹H NMR (400 MHz, DMSO) δ 13.46 (s, 1H), 9.56 (s, 1H), 8.23 (d, J = 8.5 Hz, 1H), 7.56 (d, J = 10.6 Hz, 1H), 4.90 (hept, J = 6.2 Hz, 1H), 1.26 (d, J = 6.3 Hz, 6H). |
| **IV-5** | -CH₂CH₂CH₂CH₃ | ¹H NMR (400 MHz, DMSO) δ 13.47 (s, 1H),δ 9.63 (s, 1H), 8.23 (d, J = 8.4 Hz, 1H), 7.58 (t, J = 10.6 Hz, 1H), 4.10 (t, J = 6.6 Hz, 2H), 1.60 (dt, J = 14.5, 6.7 Hz, 2H), 1.44 - 1.31 (m, 2H), 0.99 - 0.85 (m, 3H). |
| **IV-6** | -CH₂CH(CH₃)₂ | ¹H NMR (400 MHz, DMSO) δ 13.49 (s, 1H), 9.65 (s, 1H), 8.23 (d, J = 8.3 Hz, 1H), 7.58 (d, J = 10.6 Hz, 1H), 3.89 (d, J = 6.6 Hz, 2H), 2.00 - 1.83 (m, 1H), 0.93 (d, J = 6.7 Hz, 6H). |
| **IV-7** | -CH(CH₃)CH₂CH₃ | |
| **IV-8** | -C(CH₃)₃ | |
| **IV-9** | -CH₂CH₂CH₂CH₂CH₃ | ¹H NMR (400 MHz, DMSO) δ 13.47 (s, 1H), 9.63 (s, 1H), 8.25 (d, J = 8.4 Hz, 1H), 7.57 (d, J = 10.5 Hz, 1H), 4.10 (t, J = 6.6 Hz, 2H), 1.63 (p, J = 6.8 Hz, 2H), 1.41 - 1.28 (m, 4H), 0.97 - 0.82 (m, 3H). |
| **IV-10** | -CH₂CH₂CH(CH₃)₂ | |
| **IV-11** | -CH₂CH(CH₃)CH₂CH₃ | |
| **IV-12** | -CH₂C(CH₃)₃ | |
| **IV-13** | -CH(CH₂CH₃)₂ | |
| **IV-14** | -CH₂CH₂CH₂CH₂CH₂CH₃ | ¹H NMR (400 MHz, DMSO) δ 13.48 (s, 1H), 9.64 (s, 1H), 8.24 (d, J = 8.3 Hz, 1H), 7.57 (d, J = 10.5 Hz, 1H), 4.09 (t, J = 6.6 Hz, 2H), 1.69 - 1.51 (m, 2H), 1.44 - 1.19 (m, 6H), 0.87 (t, J = 6.8 Hz, 3H). |
| **IV-15** | -CH₂CH₂CH₂CH(CH₃)₂ | |
| **IV-16** | -CH₂CH(CH₂CH₃)₂ | |
| **IV-17** | -CH₂CH(CH₃)CH₂CH₂CH₃ | |
| **IV-18** | -CH(CH₂CH₃)CH₂CH₂CH₃ | |
| **IV-19** | -CH(CH₃)CH₂CH₂CH₂CH₃ | |
| **IV-20** | -CH(CH₃)CH₂CH(CH₃)₂ | |
| **IV-21** | -(CH₂)₆CH₃ | ¹H NMR (400 MHz, DMSO) δ 13.48 (s, 1H), 9.64 (s, 1H), 8.24 (d, J = 8.3 Hz, 1H), 7.57 (d, J = 10.5 Hz, 1H), 4.09 (t, J = 6.6 Hz, 2H), 1.70 - 1.49 (m, 2H), 1.30 (dd, J = 16.0, 7.4 Hz, 8H), 0.86 (t, J = 6.7 Hz, 3H). |
| **IV-22** | -CH(CH₃)(CH₂)₄CH₃ | |
| **IV-23** | -(CH₂)₇CH₃ | |
| **IV-24** | -CH(CH₃)(CH₂)₅CH₃ | |
| **IV-25** | CH₂CH(CH₂CH₃)(CH₂)₃CH₃ | |
| **IV-26** | -(CH₂)₈CH₃ | |
| **IV-27** | -(CH₂)₉CH₃ | |
| **IV-28** | -CF₃ | |
| **IV-29** | -CHF₂ | |
| **IV-30** | -CH₂CF₃ | |
| **IV-31** | -CH₂CHF₂ | |
| **IV-32** | -CH₂CH₂F | |
| **IV-33** | -CH₂CH₂Cl | ¹H NMR (400 MHz, DMSO) δ 13.52 (s, 1H), 9.89 (s, 1H), 8.24 (d, J = 8.4 Hz, 1H), 7.59 (d, J = 10.5 Hz, 1H), 4.47 - 4.34 (m, 2H), 3.96 - 3.83 (m, 2H). |
| **IV-34** | -CH₂CH₂Br | |
| **IV-35** | -CHClCH₃ | |
| **IV-36** | 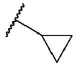 | |
| **IV-37** | 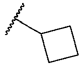 | |
| **IV-38** | 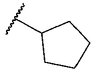 | ¹H NMR (400 MHz, DMSO) δ 13.46 (s, 1H), 9.55 (s, 1H), 8.23 (d, J = 8.3 Hz, 1H), 7.56 (d, J = 10.5 Hz, 1H), 5.18 - 5.02 (m, 1H), 2.01 - 1.78 (m, 2H), 1.76 - 1.47 (m, 6H). |
| **IV-39** | 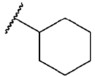 | |
| **IV-40** | 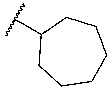 | |
| **IV-41** | 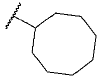 | |
| **IV-42** | 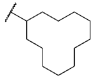 | |
| **IV-43** | 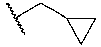 | |
| **IV-44** | 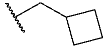 | |
| **IV-45** | 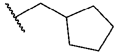 | |
| **IV-46** | 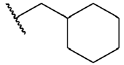 | |
| **IV-47** | 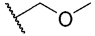 | |
| **IV-48** | 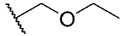 | |
| **IV-49** | 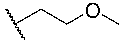 | |
| **IV-50** | 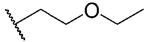 | |
| **IV-51** | 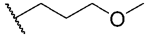 | |
| **IV-52** | 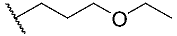 | |
| **IV-53** | 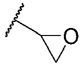 | |
| **IV-54** | 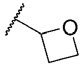 | |
| **IV-55** | 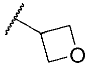 | |
| **IV-56** | 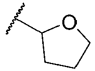 | |
| **IV-57** | 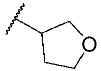 | |
| **IV-58** | 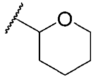 | |
| **IV-59** | 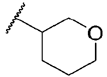 | |
| **IV-60** | 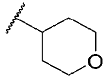 | |
| **IV-61** | 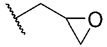 | |
| **IV-62** | 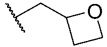 | |
| **IV-63** | 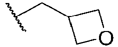 | |
| **IV-64** | 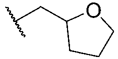 | |
| **IV-65** | 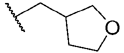 | |
| **IV-66** | 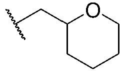 | |
| **IV-67** | 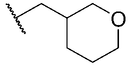 | |
| **IV-68** | 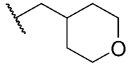 | |
| **IV-69** | -Ph | ¹H NMR (400 MHz, DMSO) δ 13.53 (s, 1H), 10.30 (s, 1H), 8.29 (d, J = 7.9 Hz, 1H), 7.64 (d, J = 10.5 Hz, 1H), 7.44 (t, J = 7.9 Hz, 2H), 7.27 (dd, J = 14.7, 7.5 Hz, 3H). |
| **IV-70** | -Bn | ¹H NMR (400 MHz, DMSO) δ 13.50 (s, 1H), 9.82 (s, 1H), 8.28 (d, J = 8.4 Hz, 1H), 7.58 (d, J = 10.5 Hz, 1H), 7.50 - 7.30 (m, 5H), 5.19 (s, 2H). |
| **IV-71** | -CH₂CH=CH₂ | ¹H NMR (400 MHz, DMSO) δ 13.51 (s, 1H), 9.78 (s, 1H), 8.26 (d, J = 8.3 Hz, 1H), 7.59 (d, J = 10.5 Hz, 1H), 5.99 (ddd, J = 22.5, 10.6, 5.4 Hz, 1H), 5.39 (dd, J = 17.2, 1.4 Hz, 1H), 5.25 (dd, J = 10.5, 1.3 Hz, 1H), 4.64 (d, J = 5.4 Hz, 2H). |
| **IV-72** | -CH₂CH=CHCH₃ | |
| **IV-73** | -CH₂CH≡CH | |

The sixth aspect of the present invention provides the use of the compound of formula (**IV**) in the prevention and control of plant diseases.

Control or controlling as used herein encompasses protective, curative and eradicative treatment of phytopathogenic fungi.

The compound of formula (**IV**) and the composition of the invention may be applied to any plants or plant parts. Plant parts are understood to mean all parts and organs of plants above and below the ground, such as shoots, leaves, needles, stalks, stems, flowers, fruit bodies, fruits, seeds, roots, tubers and rhizomes. The plant parts also include harvested material and vegetative and generative propagation material, for example cuttings, tubers, rhizomes, slips and seeds.

The plant diseases that can be prevented and controlled according to the present invention include:
Rice diseases: blast (*Magnaporthe grisea*), brown spot (*Cochliobolus miyabeanus*), sheath blight (*Rhizoctonia solani*), and bakanae disease (*Gibberella fujikuroi*), rice false smut (*Ustilaginoidea virens* (Cke,)Tak.) ;
Wheat diseases: powdery mildew (*Erysiphe graminis*), *fusarium* blight (*Fusarium graminearum, F. avenaceum, F. culmorum, Microdochium nivale*), rust (*Puccinia striiformis, P. graminis, P. recondita*), snow mould (*Micronectriella nivale*), typhulasnow blight (*Typhula* sp.), loose smut (*Ustilago tritici*), stinking smut (*Tilletia caries*), eyespot (*Pseudocercosporella herpotrichoides*), leaf blotch (*Mycosphaerella graminicola*), glume blotch (*Stagonospora nodorum*), tan spot *(Pyrenophora tritici-repentis);*
Barley diseases: powdery mildew (*Erysiphe graminis*), *fusarium* blight (*Fusarium graminearum, F. avenaceum, F. culmorum, Microdochium nivale*), rust (*Puccinia striiformis, P. graminis, P. hordei*), loose smut (*Ustilago nuda*), scald (*Rhynchosporium secalis*), net blotch (*Pyrenophora teres*), spot blotch (*Cochliobolus sativus*), leaf stripe (*Pyrenophora graminea*)*,* and *rhizoctonia* seeding blight (*Rhizoctonia solani*);
Corn diseases: smut (*Ustilago maydis*), southern leaf blight (*Cochliobolus heterostrophus*), Zonate leaf spot (*Gloeocercospora sorghi*), southern rust (*Puccinia polysora*)*,* gray leaf spot (*Cercospora zeae-maydis*), and *rhizoctonia* seeding blight (*Rhizoctonia solani*);
Citrus diseases: melanose (*Diaporthe citri*), scab (*Elsinoe fawcetti*), fruit rot (*Penicillium digitatum, P. italicum*); *Phytophthora* diseases (*Phytophthora parasitica, Phytophthora citrophthora*);
Apple diseases: blossom blight (*Monilinia mali*), canker (*Valsa ceratosperma*)*,* powdery mildew (*Podosphaera leucotricha*), *alternaria* leaf spot (*Alternaria alternata* apple pathotype), scab (*Venturia inaequalis*), bitter rot (*Colletotrichum acutatum*), and crown rot (*Phytophthora cactorum*);
Pear diseases: scab (*Venturia nashicola, V. pirina*), black spot (*Alternaria alternata* Japanese pear pathotype), rust (*Gymnosporangium haraeanum*), and *phytophthora* fruit rot (*Phytophthora cactorum*);
Peach diseases: brown rot (*Monilinia fructicola*), scab (*Cladosporium carpophilum*) and *Phomopsis* rot (*Phomopsis* sp.);
Grapes diseases: anthracnose (*Elsinoe ampelina*), ripe rot (*Glomerella cingulata*), powdery mildew (*Uncinula necator*), rust (*Phakopsora ampelopsidis*), black rot (*Guignardia bidwellii*), and downy mildew (*Plasmopara viticola*);
Diseases of Japanese persimmon: anthracnose (*Gloeosporium kaki*), and leaf spot (*Cercospora kaki, Mycosphaerella nawae*);
Diseases of gourd family: anthracnose (*Colletotrichum lagenarium*), powdery mildew (*Sphaerotheca fuliginea*), vine blight (*Mycosphaerella melonis*), *fusarium* wilt (*Fusarium oxysporum*), downy mildew (*Pseudoperonospora cubensis*), *phytophthora* rot (*Phytophthora* sp.), and damping-off (*Pythium* sp.);
Tomato diseases: early blight (*Alternaria solani*), leaf mold (*Cladosporium fulvum*), and late blight (*Phytophthora infestans*);
Eggplant disease: brown spot (*Phomopsis vexans*), and powdery mildew (*Erysiphe cichoracearum*);
Diseases of Cruciferous Vegetables: *alternaria* leaf spot (*Alternaria japonica*), white spot (*Cercosporella brassicae*), clubroot (*Plasmodiophora brassicae*), and downy mildew (*Peronospora parasitica*);
Welsh onion diseases: rust (*Puccinia allii*) and downy mildew (*Peronospora destructor*);
Soybean diseases: purple stain (*Cercospora kikuchii*), sphaceloma scad (*Elsinoe glycines*), pod and stem blight (*Diaporthe phaseclorum* var. *sojae*), *septoria* brown spot (*Septoria glycines*), frog eye leaf spot (*Cercospora sojina*), rust (*phakopsora pachyrhizi*), *phytophthora* root and stem rot (*Phytophthora sojae*), *rhizoctonia* aerial blight (*Rhizoctonia solani*), target spot (*Corynespora cassiicola*), and *sclerotinia* stem rot (*Sclerotinia sclerotiorum*);
Kidney bean diseases: anthracnose (*Colletotrichum lindemthianum*);
Peanut diseases: early leaf spot (*Cercospora personata*), late leaf spot (*Cercospora arachidicola*), and southern blight (*Sclerotium rolfsii*);
Garden pea diseases: powdery mildew (*Erysiphe pisi*);
Potato diseases: early blight (*Alternaria solani*), late blight (*Phytophthora infestans*), Pink rot (*Phytophthora Erythroseptica*), and powdery scab (*Spongospora subterranean* f. sp. *subterranea;*
Strawberry diseases: powdery mildew (*Sphaerotheca humuli*), and anthracnose (*Glomerella cingulata*);
Tea diseases: net blister blight (*Exobasidium reticulatum*), white scab (*Elsinoe leucospila*), gray blight (*Pestalotiopsis* sp.) and anthracnose (*Colletotrichum theae-sinensis*);
Tobacco diseases: brown spot (*Alternaria longipes*), powdery mildew (*Erysiphe cichoracearum*), anthracnose (*Colletotrichum tabacum*), downy mildew (*Peronospora tabacina*), and black shank (*Phytophthora nicotianae*);
Rapeseed diseases: *sclerotinia* rot (*Sclerotinia sclerotiorum*), and *rhizoctonia* seeding blight (*Rhizoctonia solani*);
Cotton diseases: *rhizoctonia* seeding blight (*Rhizoctonia solani*);
Sugar beet diseases: *cercospora* leaf spot (*Cercospora beticola*), leaf blight (*Thanatephorus cucumeris*), root rot (*Thanatephorus cucumeris*), and *aphanomyces* root rot (*Aphanomyces cochlioides*);
Rose diseases: black spot (*Diplocarpon rosae*), powdery mildew (*Sphaerotheca pannosa*), and downy mildew (*Peronospora sparsa*);
Diseases of *Chrysanthemum:* downy mildew (*Bremia lactucae*)*,* leaf blight (*Septoria chrysanthemi-indici*), and white rust (*Puccinia horiana*), leaf blight (*Septoria chrysanthemi-indici*), and white rust (*Puccinia horiana*);
Various crops diseases: diseases caused by *Pythium* spp. (*Pythium aphanidermatum, Pythium debarianum, Pythium irregulare,* and *Pythium ultimum*), gray mold (*Botrytis cinerea*), and *sclerotinia* rot; *Sclerotinia scleroticrum*);
Diseases of Japanese radish: *alternaria* leaf spot (*Alternaria brassicicola*);
Turfgrass diseases: dollar spot (*Sclerotinia* homeocarpa), brown patch and large patch (*Rhizoctonia solani*);
Banana diseases: Sigatoka disease (*Mycosphaerella fijiensis, Mycosphaerella musicola*);
seed diseases or diseases in the early stages of the growth of various plants caused by *Aspergiilus* spp., *Penicillium* spp., *Fusarium* spp., *Gibberella* spp., *Trichoderma*
*spp., Thielaviopsis* spp., *Rhizopus* spp., *Mucor* spp., *Corticium* spp., *Phoma* spp., *Rhizoctonia spp.* or *Diplodia* spp.; and viral diseases of various plants mediated by *Polymyxa spp.* or *Olpidium* spp.; and so on.

The seventh aspect of the present invention provides a fungicidal composition comprising, as an active substance, a compound of formula (IV) or an agriculturally acceptable salt thereof, and an agriculturally acceptable carrier, wherein the active substance is present in an amount of 1-99 wt%. Generally, a compound as general formula (**IV**) is used in the form of a composition (e.g. formulation) containing a carrier. A compound as general formula (**IV**) and compositions thereof can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ulv) liquid, ultra low volume (ulv) suspension, water dispersible granules or tablets, water dispersible powder for slurry treatment, water soluble granules or tablets, water soluble powder for seed treatment and wettable powder.

A formulation typically comprises a liquid or solid carrier and optionally one or more customary formulation auxiliaries, which may be solid or liquid auxiliaries, for example unepoxidized or epoxidized vegetable oils (for example epoxidized coconut oil, rapeseed oil or soya oil), antifoams, for example silicone oil, preservatives, clays, inorganic compounds, viscosity regulators, surfactant, binders and/or tackifiers. The composition may also further comprise a fertilizer, a micronutrient donor or other preparations which influence the growth of plants as well as comprising a combination containing the compound of the invention with one or more other biologically active agents, such as bactericides, fungicides, nematicides, plant activators, acaricides, and insecticides. The compositions are prepared in a manner known per se, in the absence of auxiliaries for example by grinding, screening and/or compressing a solid compound of the present invention and in the presence of at least one auxiliary for example by intimately mixing and/or grinding the compound of the present invention with the auxiliary (auxiliaries). In the case of solid compounds of the invention, the grinding/milling of the compounds is to ensure specific particle size.

Examples of compositions for use in agriculture are emulsifiable concentrates, suspension concentrates, microemulsions, oil dispersibles, directly sprayable or dilutable solutions, spreadable pastes, dilute emulsions, soluble powders, dispersible powders, wettable powders, dusts, granules or encapsulations in polymeric substances, which comprise-at least-a compound as general formula (**IV**) and the type of composition is to be selected to suit the intended aims and the prevailing circumstances.

As a rule, the compositions comprise 0.1 to 99%, especially 0.1 to 95%, of compound as general formula (**IV**) and 1 to 99.9%, especially 5 to 99.9%, of at least one solid or liquid carrier, it being possible as a rule for 0 to 25%, especially 0.1 to 20%, of the composition to be surfactants (% in each case meaning percent by weight). Whereas concentrated compositions tend to be preferred for commercial goods, the end consumer as a rule uses dilute compositions which have substantially lower concentrations of active ingredient.

Examples of foliar formulation types for pre-mix compositions are:
GR: Granules
WP: wettable powders
WG: water-dispersible granules (powders)
SG: water soluble granules
SL: soluble concentrates
EC: emulsifiable concentrate
EW: emulsions, oil in water
ME: micro-emulsion
SC: aqueous suspension concentrate
CS: aqueous capsule suspension
OD: oil-based suspension concentrate, and
SE: aqueous suspo-emulsion.

Whereas, examples of seed treatment formulation types for pre-mix compositions are:
WS: wettable powders for seed treatment slurry
LS: solution for seed treatment
ES: emulsions for seed treatment
FS: suspension concentrate for seed treatment
WG: water dispersible granules, and
CS: aqueous capsule suspension.

Examples of formulation types suitable for tank-mix compositions are solutions, dilute emulsions, suspensions, or a mixture thereof, and dusts.

As with the nature of the formulations, the methods of application, such as foliar, drench, spraying, atomizing, dusting, scattering, coating or pouring, are chosen in accordance with the intended objectives and the prevailing circumstances.

The tank-mix compositions are generally prepared by diluting with a solvent (for example, water) the one or more pre-mix compositions containing different pesticides, and optionally further auxiliaries.

Suitable carriers and adjuvants can be solid or liquid and are the substances ordinarily employed in formulation technology, e.g. natural or regenerated mineral substances, solvents, dispersants, wetting agents, tackifiers, thickeners, binders or fertilizers.

Generally, a tank-mix formulation for foliar or soil application comprises 0.1 to 20%, especially 0.1 to 15%, of the desired ingredients, and 99.9 to 80%, especially 99.9 to 85%, of a solid or liquid auxiliaries (including, for example, a solvent such as water), where the auxiliaries can be a surfactant in an amount of 0 to 20%, especially 0.1 to 15%, based on the tank-mix formulation.

Typically, a pre-mix formulation for foliar application comprises 0.1 to 99.9%, especially 1 to 95%, of the desired ingredients, and 99.9 to 0.1%, especially 99 to 5%, of a solid or liquid adjuvant (including, for example, a solvent such as water), where the auxiliaries can be a surfactant in an amount of 0 to 50%, especially 0.5 to 40%, based on the pre-mix formulation.

Normally, a tank-mix formulation for seed treatment application comprises 0.25 to 80%, especially 1 to 75%, of the desired ingredients, and 99.75 to 20%, especially 99 to 25%, of a solid or liquid auxiliaries (including, for example, a solvent such as water), where the auxiliaries can be a surfactant in an amount of 0 to 40%, especially 0.5 to 30%, based on the tank-mix formulation.

Typically, a pre-mix formulation for seed treatment application comprises 0.5 to 99.9%, especially 1 to 95%, of the desired ingredients, and 99.5 to 0.1%, especially 99 to 5%, of a solid or liquid adjuvant (including, for example, a solvent such as water), where the auxiliaries can be a surfactant in an amount of 0 to 50%, especially 0.5 to 40%, based on the pre-mix formulation.

Whereas commercial products will preferably be formulated as concentrates (e.g., pre-mix composition (formulation)), the end user will normally employ dilute formulations (e.g., tank mix composition).

Preferred seed treatment pre-mix formulations are aqueous suspension concentrates. The formulation can be applied to the seeds using conventional treating techniques and machines, such as fluidized bed techniques, the roller mill method, rotostatic seed treaters, and drum coaters. Other methods, such as spouted beds may also be useful. The seeds may be presized before coating. After coating, the seeds are typically dried and then transferred to a sizing machine for sizing. Such procedures are known in the art. The compounds of the present invention are particularly suited for use in soil and seed treatment applications.

The compositions have a broad range of efficacy as a fungicide. The exact amount of the composition to be applied is dependent not only on the relative amounts of the components, but also on the particular action desired, the fungal species to be controlled, and the stage of growth thereof, as well as the part of the plant or other product to be contacted with the composition. Thus, formulations containing the composition may not be equally effective at similar concentrations or against the same fungal species.

The eighth aspect of the present invention provides a method of preventing or controlling the plant disease using a fungicidal composition, wherein the composition having the compound of general formula (**IV**) as an active substance is applied to a plant, thereby treating the plant to resist infection by fungal species.

The present compositions can be applied to fungi or their locus by the use of conventional ground sprayers, granule applicators, and by other conventional means known to those skilled in the art.

Further, the present invention provides a method of preventing or controlling a plant disease using a fungicidal composition as described above, wherein an effective amount of the composition is applied to a plant, thereby treating said plant to resist infection by a fungal species, said composition as described above.

Fungal species including fungi that are resistant to other fungicides. Fungi that are "resistant" to particular fungicides refer e.g. to strains that are less sensitive to that fungicide compared to the expected sensitivity of the same species. The expected sensitivity can be measured using e.g. a strain that has not previously been exposed to the fungicide.

Application according to the methods or uses according to the above is preferably to a crop of plants, the locus thereof or propagation material thereof. Preferably application is to the locus of the plant or the propagation material of the plant, more preferably to the propagation material. Application of the compounds according to general formula (**IV**) can be performed according to any of the usual modes of application, e.g. foliar, drench, soil, in furrow, seed treatment etc.

The compounds as defined by general formula (**IV**) are preferably used for pest control at 1 to 500 g/ha, preferably 50-200 g/ha.

The compounds as defined by general formula (**IV**) are suitable for use on any vegetable, tomato and potato plant, including those that have been genetically modified to be resistant to active ingredients such as herbicides, or to produce biologically active compounds that control infestation by plant pests.

Plants and plant cultivars which may be treated by the above disclosed methods include plants and plant cultivars which are hybrid plants that already express the characteristic of heterosis or hybrid vigor which results in generally higher yield, vigor, health and resistance towards biotic and abiotic stresses.

The present disclosure includes within its scope methods for the control or prevention of fungal attack. These methods comprise applying to the locus of the fungus, or to a locus in which the infestation is to be prevented (for example applying to wheat or barley plants), a fungicidally effective amount of the composition of the present invention. The composition of the present invention is suitable for treatment of various plants at fungicidal levels, while exhibiting low phytotoxicity. The composition of the present invention is useful in a protectant or eradicant fashion. The composition of the present invention is applied by any of a variety of known techniques, either as the composition of the present invention or as a formulation comprising the composition of the present invention. For example, the composition of the present inventions may be applied to the roots, seeds or foliage of plants for the control of various fungi, without damaging the commercial value of the plants. The composition of the present invention is applied in the form of any of the generally used formulation types, for example, as solutions, dusts, wettable powders, flowable concentrates, or emulsifiable concentrates. These materials are conveniently applied in various known fashions.

The control method of the present invention can control plant diseases by applying the composition of the present invention to plants or soil for cultivating plants.

The method for applying the composition of the present invention is not particularly limited, as far as the applying form is a form by which the present compound may be applied substantially, and includes, for example, an application to plants such as a foliar application; an application to area for cultivating plants such as a submerged treatment; and an application to seed such as seed disinfection.

The application dose of the composition of the present invention varies depending on weather conditions, dosage forms, timing of application, methods of application, areas to be applied, target diseases, target crops and the others, and is in the range of usually from 1 to 500 g, and preferably from 2 to 200 g per 1,000 m² of the area to be applied. The emulsifiable concentrate, the wettable powder or the suspension concentrate etc., is usually applied by diluting it with water. In this case, the concentration of the composition of the present invention after dilution is in the range of usually 0.0005 to 2% by weight, and preferably 0.005 to 1% by weight, and the dust formulation or the granular formulation etc., is usually applied as itself without diluting it. In the application to seeds, the amount of the composition of the present invention is in the range of usually from 0.001 to 100 g, and preferably from 0.01 to 50 g per 1 kg of the seeds.

Examples of the place where the plant diseases grow include paddy fields, fields, tea gardens, orchards, non-agricultural lands, houses, nursery trays, nursery boxes, nursery soils and nursery bed.

The composition of the present invention can be used as agent for controlling plant disease in agricultural lands such as fields, paddy fields, lawns, and orchards. The composition of the present invention can control diseases occurred in the agricultural lands or the others for cultivating the following "plant" and the others.

The "plant" in the present invention thereof include, without limitation:
Vegetable or vegetable plants include, without limitation, okra, spinach, lettuce, asparagus, cabbages, carrots, onions, peppers, hot peppers, bell peppers, cucumbers, corn, lettuce, asparagus, Chinese cabbages, tomatoes, squashes, eggplants and beets;
Fruits or fruit plants: include, without limitation, grapes, apples, pears, peaches, hawthorns, persimmons, jujubes, citrus, cherries, strawberries, blueberries, oranges, lemons, grapefruit, plums, apricots, bananas, sugarcane and lychees;
Grain or grain plants: include, without limitation, wheat, barley, rye, rice, oats, sorghum, soybeans and potatoes;
Oilseed crops: rape, mustard, poppy, olive, sunflower, coconut, castor oil plant, cocoa and peanuts;
Other plants: turf, tobacco, nuts, coffee, tea, pepper, grapevines, hops and latex plants;
Ornamental plants: flowers, shrubs, larch trees, and coniferous trees.

The composition of the present invention can be used to protect plants from plant diseases.

According to the invention, the compounds as defined by general formula (**IV**) can be advantageously used to treat transgenic plants, plant cultivars or plant parts that received genetic material which imparts advantageous and/or useful properties (traits) to these plants, plant cultivars or plant parts. Therefore, it is contemplated that the present invention may be combined with one or more recombinant traits or transgenic event(s) or a combination thereof. For the purposes of this application, a transgenic event is created by the insertion of a specific recombinant DNA molecule into a specific position (locus) within the chromosome of the plant genome. The insertion creates a novel DNA sequence referred to as an "event" and is characterized by the inserted recombinant DNA molecule and some amount of genomic DNA immediately adjacent to/flanking both ends of the inserted DNA. Such trait(s) or transgenic event(s) include, but are not limited to, pest resistance, water use efficiency, yield performance, drought tolerance, seed quality, improved nutritional quality, hybrid seed production, and herbicide tolerance, in which the trait is measured with respect to a plant lacking such trait or transgenic event. Concrete examples of such advantageous and/or useful properties (traits) are better plant growth, vigor, stress tolerance, standability, lodging resistance, nutrient uptake, plant nutrition, and/or yield, in particular improved growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processability of the harvested products, and increased resistance against animal and microbial pests, such as against insects, arachnids, nematodes, mites, slugs and snails.

Among DNA sequences encoding proteins which confer properties of tolerance to such animal and microbial pests, in particular insects, mention will particularly be made of the genetic material from Bacillus thuringiensis encoding the Bt proteins widely described in the literature and well known to those skilled in the art. Mention will also be made of proteins extracted from bacteria such as Photorhabdus (WO97/17432 and WO98/08932). In particular, mention will be made of the Bt Cry or VIP proteins which include the CrylA, CryIAb, CryIAc, CryIIA, CryIIIA, CryIIIB2, Cry9c Cry2Ab, Cry3Bb and CryIF proteins or toxic fragments thereof and also hybrids or combinations thereof, especially the CrylF protein or hybrids derived from a CrylF protein (e.g. hybrid CrylA-CrylF proteins or toxic fragments thereof), the CrylA-type proteins or toxic fragments thereof, preferably the CrylAc protein or hybrids derived from the CrylAc protein (e.g. hybrid CrylAb-CrylAc proteins) or the CrylAb or Bt2 protein or toxic fragments thereof, the Cry2Ae, Cry2Af or Cry2Ag proteins or toxic fragments thereof, the CrylA.105 protein or a toxic fragment thereof, the VIP3Aa19 protein, the VIP3Aa20 protein, the VIP3A proteins produced in the COT202 or COT203 cotton events, the VIP3Aa protein or a toxic fragment thereof as described in Estruch et al. (1996), Proc Natl Acad Sci USA. 28; 93(11):5389-94, the Cry proteins as described in WO2001/47952, the insecticidal proteins from Xenorhabdus (as described in WO98/50427), Serratia (particularly from S. entomophila) or Photorhabdus species strains, such as Tc-proteins from Photorhabdus as described in WO98/08932. Also any variants or mutants of any one of these proteins differing in some amino acids (1-10, preferably 1-5) from any of the above named sequences, particularly the sequence of their toxic fragment, or which are fused to a transit peptide, such as a plastid transit peptide, or another protein or peptide, is included herein.

### DETAILED DESCRIPTION OF THE INVENTION

Hereinafter, the present invention will be described with reference to the following but not limiting examples. Any simple replacement or improvement made to the present invention by the person skilled in the art shall fall within the protection scope of the present invention.

Several methods for preparing compounds of the present invention are explained in detail in the following schemes and examples. The raw materials can be purchased from the market or prepared using methods known in the literature or as detailed in the explanation. The person skilled in the art should understand that the compounds of the present invention can also be synthesized using other synthesis routes. Although specific raw materials and conditions in the synthesis route have been explained below, they can be easily replaced with other similar raw materials and conditions, and various isomers of the compounds prepared by variants or variations of the preparation method of the present invention shall fall within the protection scope of the present invention. In addition, the preparation method described below can be further modified according to the disclosure of the present invention, using conventional chemical methods well-known to the person skilled in the art, for example, protecting appropriate groups during the reaction process, and so on.

The method examples provided below are intended to promote further understanding of the preparation method of the present invention, and the specific substances, types, and conditions used are determined as further explanations of the present invention, not as limitations to the reasonable protection scope of the present invention. The raw materials and reagents used in the synthesis of the compounds described below can either be purchased from the market or easily prepared by the person skilled in the art.

The abbreviations used in the examples have the following meanings:
THF: Tetrahydrofuran
DMF: N,N-dimethylformamide
DMAC: N,N-dimethylacetamide
NMP: N-methylpyrrolidone
DMAP: 4-dimethylaminopyridine
1,2-DCE: 1,2-dichloroethane

The analytical instruments described in the examples are as follows:
1. High performance liquid chromatography (hereinafter referred to as HPLC):
   Method A:
      Using Agilent Technologies, 1260 Infinity II device
      Column: Agilent Eclipse Plus C18 3.5 µm, 4.6*100 mm
      Mobile phase: A: water+0.1% phosphoric acid; B: acetonitrile, temperature: 30°C
      Gradient: 5% B to 95% B within 15 min; 95% B 3 min
      Flow rate: 1 mL/min
   Method B:
      Using Agilent Technologies, 1260 Infinity II device
      Column: Agilent Eclipse Plus C18 3.5 µ m, 4.6*100 mm
      Mobile phase: A: water+0.1% phosphoric acid; B: methanol, temperature: 30°C
      Gradient: 30% B to 95% B within 20 min; 95% B 3 min
      Flow rate: 1 mL/min
2. Ultra high performance liquid chromatography-tandem mass spectrometry (hereinafter referred to as
   LC-MS): using Waters, ACQUITY H-Class UPLC-SQ Detector 2 device
   Column: ACQUITY UPLC ^{®} BEH C18 1.7 µm, 2.1*50 mm Column
   Mobile phase: A: water + 0.2% formic acid; b: acetonitrile, temperature: 30°C
   Gradient: 10% B to 95% B within 5 min; 95% B 1 min
   Flow rate: 0.5 mL/min.
   MS method: ESI positive, negative, mass range (m/z): 100-800
3. Gas chromatograph (hereinafter referred to as GC): using Agilent Technologies, 7890B GC device
   Detector: FID
   Chromatographic column: HP-1 30 m*530 µm*10.5 µm
   Injection port temperature: 250°C
   Split ratio: 40:1
   Flow rate: 20 mL/min
   H₂: 30 mL/min
   Air: 300 mL/min
   He: 25 mL/min
   Detector temperature: 280°C
   Method: remain the temperature of 40°C for 2min, raise the temperature to 260°C at a rate of 20°C/min, and remain the temperature of 260°C for 5 min, a total time of 18 min
4. Gas chromatography-tandem mass spectrometry (hereinafter referred to as GC-MS): using Agilent Technologies, 7890B GC System-5977A MSD device
   Column: Agilent Technologies, HP-5MS UI 0.25 µm, 30m*0.250mm
   Sample injector temperature: 250°C
   Chromatographic column flow rate: helium 1 mL/min
   Method: remain the temperature of 40°C for 2 min, raise the temperature to 280°C at a rate of 20°C/min, remain the temperature of 280°C for 5 min, a total time of 19 min
   MSD transmission line temperature: 280°C
   EI ion source temperature: 230°C, MS quadrupole temperature: 150°C, scanning range: 30.00-400.00

In addition, the chemical shift value of proton nuclear magnetic resonance spectrum (hereinafter referred to as ¹H-NMR) recorded below is measured at 400MHz (Bruker, AVANCE III HD 400M) in deuterated chloroform solvent using Me₄Si (tetramethylsilane) as the standard substance. In the case of determination in deuterated dimethyl sulfoxide solvent, it is shown as "(DMSO-d6)" in the data of chemical shift value. It should be noted that the symbols in the chemical shift values of ¹H-NMR represent the following meanings.

s: singlet, d: doublet, dd: double doublet, dt: double triplet, td: triple doublet, ddd: double double doublet, t: triplet, q: quartet, sep: septet, m: multiplet, brs: broad singlet. In addition, in the presence of two or more stereoisomers, each chemical shift value is marked with "and" for signals that can be analyzed.

The examples of representative reaction operations are as follows, and other reaction operations or the synthesis of other compounds can be successfully carried out by a similar method, which will not be explained in detail here.

### Example 1: Synthesis of compound IV-1

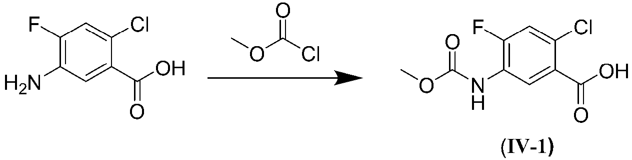

In a 2 L four-necked flask, were placed 189.57 g (1.00 mol) of 2-chloro-4-fluoro-5-aminobenzoic acid and 800 g of THF while stirring to dissolve. The mixture was heated to reflux (69°C), and 113.40 g (1.20 mol) of methyl chloroformate was slowly added dropwise within 2 h. After the dropwise addition was completed, reflux reaction was continued for 4 h, and the reaction was completed when the raw material was less than 0.5% by HPLC detection. The reaction mixture was distilled under reduced pressure to remove the solvent to give 243.15 g of white solid of compound **IV-1,** with an HPLC area normalization purity of 98.8% and a yield of 98.2%. ¹H NMR (400 MHz, DMSO-*d*₆) δ 13.49 (s, 1H), 9.68 (s, 1H), 8.22 (d, *J* = 8.5 Hz, 1H), 7.58 (d, *J =* 10.6 Hz, 1H), 3.69 (s, 3H).

### Example 2: Synthesis of compound IV-2

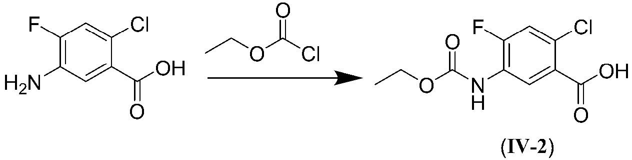

In a 2 L four-necked flask, were placed 189.57 g (1.00 mol) of 2-chloro-4-fluoro-5-aminobenzoic acid and 800 g of THF while stirring to dissolve. The mixture was heated to reflux (69°C), and 130.22 g (1.20 mol) of ethyl chloroformate was slowly added dropwise within 2 h. After the dropwise addition was completed, reflux reaction was continued for 4 h, and the reaction was completed when the raw material was less than 0.5% by HPLC detection. The reaction mixture was distilled under reduced pressure to remove the solvent to give 254.04 g of white solid of compound **IV-2,** with an HPLC area normalization purity of 98.2% (Method B) and a yield of 97.1 %.

### Example 3: Synthesis of compound IV-2

In a 2 L four-necked flask, were placed 189.57 g (1.00 mol) of 2-chloro-4-fluoro-5-aminobenzoic acid and 800 g of 2-methyltetrahydrofuran while stirring to dissolve. The mixture was heated to reflux (81°C), and 130.23 g (1.20 mol) of ethyl chloroformate was slowly added dropwise within 2 h. After the dropwise addition was completed, reflux reaction was continued for 4 h, and the reaction was completed when the raw material was less than 0.5% by HPLC detection. The reaction mixture was distilled under reduced pressure to remove the solvent to give 253.78 g of white solid of compound **IV-2,** with an HPLC area normalization purity of 98.3% (Method B) and a yield of 97.0%.

Similarly, compounds **IV-3** to **IV-73** can be prepared according to the method of examples 1-3, and some examples were shown in Table 3:

**Table 3**

| NO. | R₃OCOCl | solvent | Temperature (°C) | yield (%) | purity (%) |
|---|---|---|---|---|---|
| **IV-3** | CH₃CH₂CH₂OCOCl | THF | 66 | 97.2 | 98.1 |
| **IV-4** | (CH₃)₂CHOCOCl | THF | 66 | 97.1 | 98.1 |
| **IV-5** | CH₃CH₂CH₂CH₂OCOCl | THF | 66 | 96.9 | 97.3 |
| **IV-6** | (CH₃)₂CHCH₂OCOCl | THF | 66 | 96.7 | 97.8 |
| **IV-9** | CH₃(CH₂)₄OCOCl | THF | 66 | 97.0 | 97.1 |
| **IV-14** | CH₃(CH₂)₅OCOCl | THF | 66 | 96.8 | 97.4 |
| **IV-21** | CH₃(CH₂)₆OCOCl | THF | 66 | 96.7 | 97.6 |
| **IV-38** | 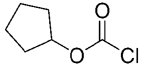 | THF | 66 | 96.2 | 97.4 |
| **IV-69** | 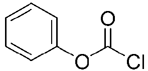 | THF | 66 | 97.0 | 98.0 |
| **IV-70** | 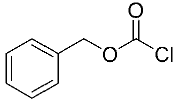 | THF | 66 | 96.5 | 97.3 |
| **IV-71** | CH₂=CHCH₂OCOCl | THF | 66 | 96.2 | 96.9 |

### Example 4: Synthesis of compound I-1

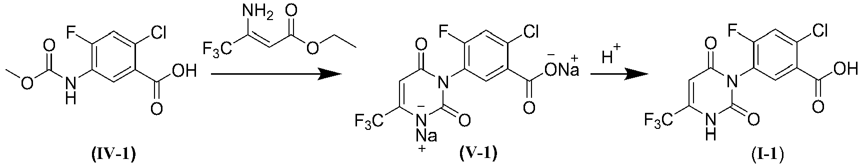

In a 2 L four-necked flask, were placed 800 g of DMAC and 247.61 g (1.00 mol) of compound **IV-1** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 329.71 g of the title compound **I-1,** with a yield of 93.5% and an HPLC area normalization purity of 98.1% (Method B).

### Example 5: Synthesis of compound I-1

In a 2 L four-necked flask, were placed 800 g of DMF and 247.61 g (1.00 mol) of compound **IV-1** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 331.12 g of the title compound **I-1,** with a yield of 93.9% and an HPLC area normalization purity of 98.4% (Method B).

### Example 6: Synthesis of compound I-1

In a 2 L four-necked flask, were placed 800 g of NMP and 247.61 g (1.00 mol) of compound **IV-1** while stirring well. At room temperature, 180.07 g (1.00 mol) of sodium methoxide-methanol solution (30%) was added dropwise, and after dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 321.95 g of the title compound **I-1,** with a yield of 91.3% and an HPLC area normalization purity of 97.5% (Method B).

### Example 7: Synthesis of compound I-2

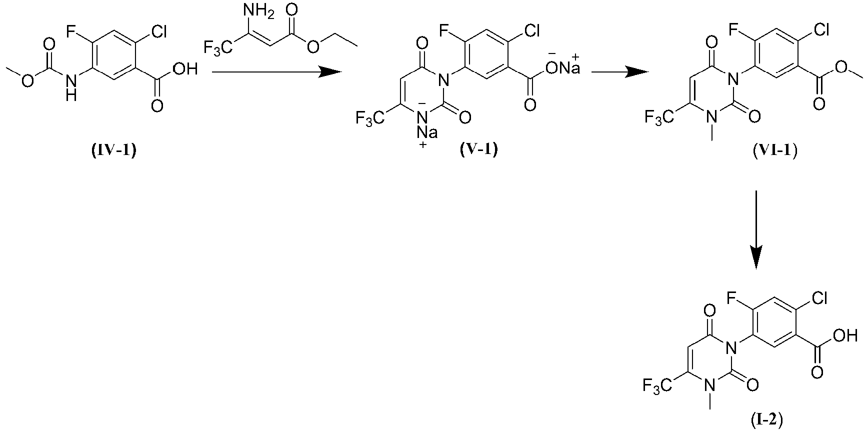

In a 2 L four-necked flask, were placed 800 g of DMF and 247.61 g (1.00 mol) of compound **IV-1** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at this temperature until there were no distilled methanol and ethanol. The reaction time was about 4 h, and the reaction was completed when the raw material was less than 0.5% by HPLC detection. The reaction mixture was cooled to 0-5°C, 378.39 g (3.00 mol) dimethyl sulfate was added dropwise within 2 h, and the reaction temperature was controlled at 0-5°C. After the dropwise addition was completed, the reaction was continued at this temperature for 2 h and then the temperature slowly rose to room temperature with much solid precipitated. After holding for 3 h, the conversion of intermediate **V-1** was basically completed, and the content of compound **VI-1** by HPLC detection was 90.9%. After adding 800 g of water and stirring for 30 min, white solid was obtained by filtration, and the filter cake was washed with water to give 445.20 g of wet product of the title compound **VI-1** with an HPLC area normalization purity of 97.7%. The wet product was directly used for subsequent steps without additional treatment.

In a 3 L four-necked reaction flask, were placed 445.20 g of the above wet product of compound **VI-1** and 1200 g of acetic acid which were heated to 100°C, and 1000 g of concentrated hydrochloric acid (36%) was slowly added dropwise with a dropwise addition time of 2 h. After the dropwise addition was completed, the reaction was continued for 8 h, and that the content of the raw material **VI-1** was less than 1.0% by sampling and detecting was considered as the end of the reaction. After the reaction was completed, the mixed solution of acetic acid and hydrochloric acid (about 1800 g) was distilled under reduced pressure. The residue was cooled to room temperature and filtered to give white solid. The filter cake was washed with water and dried to give 303.59 g of the title compound **I-2,** with a three-step yield of 82.8% and an HPLC area normalization purity of 98.2% (Method B). ¹H NMR (400 MHz, DMSO-d6) δ 13.72 (s, 1H), 8.05 (d, J = 7.9 Hz, 1H), 7.83 (d, J = 9.6 Hz, 1H), 6.60 (s, 1H), 3.42 (s, 3H).

### Example 8: Synthesis of compound I-1

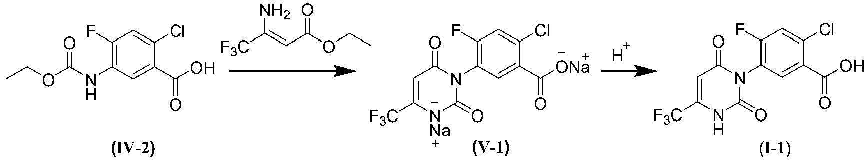

In a 2 L four-necked flask, were placed 800 g of DMAC and 261.63 g (1.00 mol) of compound **IV-2** (prepared according to the method in example 1) while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was performed at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 326.54 g of the title compound **I-1,** with a yield of 92.6% and an HPLC area normalization purity of 98.1% (Method B). ¹H NMR (400 MHz, DMSO-*d*₆) δ 13.68 (s, 1H), 12.83 (s, 1H), 8.09 (d, *J =* 7.9 Hz, 1H), 7.82 (d, *J =* 9.6 Hz, 1H), 6.45 (s, 1H).

### Example 9: Synthesis of compound I-1

In a 2 L four-necked flask, were placed 800 g of DMF and 261.63 g (1.00 mol) of compound **IV-2** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was performed at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 329.36 g of the title compound **I-1,** with a yield of 93.4% and an HPLC area normalization purity of 98.6% (Method B).

### Example 10: Synthesis of compound I-1

In a 2 L four-necked flask, were placed 800 g of NMP and 261.63 g (1.00 mol) of compound **IV-2** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was performed at 90-100°C until there were no distilled methanol and ethanol. The reaction time was about 4 h, and the reaction was completed when the raw material was less than 0.5% by HPLC detection. Most of the solvent was removed by reduced pressure distillation, 1000 g of water was added while stirring, and the pH value was adjusted to 1-2 by adding concentrated hydrochloric acid (36%) dropwise. A large amount of white solid was precipitated during the dropwise addition, and the white solid was obtained by filtration. The filter cake was washed with water and dried to give 322.30 g of the title compound **I-1,** with a yield of 91.4% and an HPLC area normalization purity of 97.3% (Method B).

### Example 11: Synthesis of compound V-1

In a 250 mL four-necked flask, were placed 60 g of DMAC and 20 g (76.4 mmol) of compound **IV-2** while stirring well. At room temperature, 7.34 g (76.4 mmol) of sodium tert-butoxide was added, and after addition, 15.38 g (84.0 mmol) of ethyl 3-amino-4,4,4-trifluorocrotonate was added. Then the mixture was heated to 90-100°C, and 11.07 g (115.2 mmol) of sodium tert-butoxide was added. After the addition was completed, the reaction was performed at 90-100°C with a reaction time of about 2 h, and the raw material was no longer reduced by HPLC detection. The HPLC qualitative yield of compound **V-1** was 86.62% (Method B).

### Example 12: Synthesis of compound V-1

In a 250 mL four-necked flask, were placed 60 g of DMAC and 20 g (76.4 mmol) of compound **IV-2** while stirring well. At room temperature, 7.88 g (197.0 mmol) of sodium hydride (60%) was added, and after addition, 15.38 g (84.0 mmol) of ethyl 3-amino-4,4,4-trifluorocrotonate was added. After the addition was completed, the reaction was performed at 90-100°C with a reaction time of about 1 h, and the raw material was no longer reduced by HPLC detection. The HPLC qualitative yield of compound **V-1** was 88.43% (Method B).

### Example 13: Synthesis of compound I-2

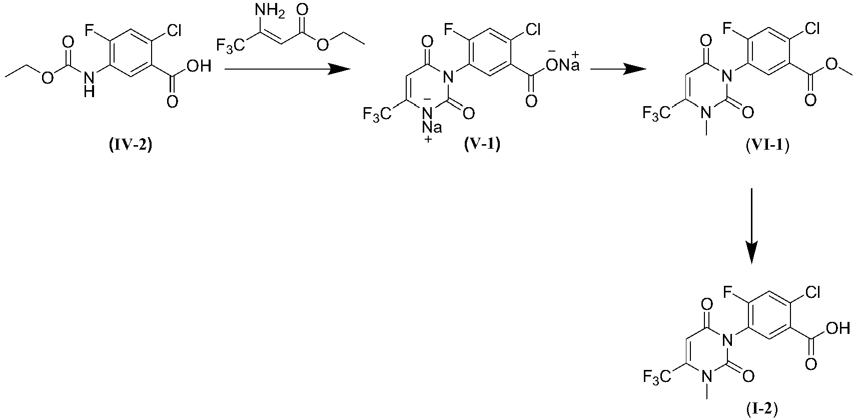

In a 2 L four-necked flask, were placed 800 g of DMF and 261.63 g (1.00 mol) of compound **IV-2** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at this temperature until there were no distilled methanol and ethanol. The reaction time was about 4 h, and the reaction was completed when the raw material was less than 0.5% by HPLC detection. The reaction mixture was cooled to 0-5°C, 378.39 g (3.00 mol) dimethyl sulfate was added dropwise within 2 h, and the reaction temperature was controlled at 0-5°C. After the dropwise addition was completed, the reaction was continued at this temperature for 2 h and then the temperature slowly rose to room temperature with much solid precipitated. After holding for 3 h, the conversion of intermediate **V-1** was basically completed, and the content of compound **VI-1** by HPLC detection was 90.3%. After adding 800 g of water and stirring for 30 min, white solid was obtained by filtration, and the filter cake was washed with water to give 437.07 g of wet product of the title compound **VI-1** with an HPLC area normalization purity of 97.7% (Method B). The wet product was directly used for subsequent reaction without additional treatment.

In a 3 L four-necked reaction flask, were placed 437.07 g of the above wet product of compound **VI-1** and 1200 g of acetic acid which were heated to 100°C, and 1000 g concentrated hydrochloric acid (36%) was slowly added dropwise with a dropwise addition time of 2 h. After the dropwise addition was completed, the reaction was continued for 8 h, and that the content of the raw material **VI-1** was less than 1.0% by HPLC detection was considered as the end of the reaction. After the reaction was completed, the mixed solution of acetic acid and hydrochloric acid (about 1800 g) was distilled under reduced pressure. The residue was cooled to room temperature and filtered to give white solid. The filter cake was washed with water and dried to give 301.02 g of the title compound **I-2,** with a three-step yield of 82.1% and an HPLC area normalization purity of 98.0% (Method B).

### Example 14: Synthesis of compound I-2

In a 2 L four-necked flask, were placed 800 g of DMF and 261.63 g (1.00 mol) of compound **IV-2** while stirring well. At room temperature, 180.07 g (1.00 mol) of sodium methoxide-methanol solution (30%) was added dropwise, and after the dropwise addition, 219.75 g (1.20 mol) of ethyl 3-amino-4,4,4-trifluorocrotonate was added. Then the mixture was heated to 90-100°C, and 198.14 g (1.10 mol) of sodium methoxide-methanol solution (30%) was slowly added dropwise within 4 h while distilling and collecting the distilled methanol and ethanol. After the dropwise addition was completed, the reaction was continued at this temperature until there were no distilled methanol and ethanol. The reaction time was about 4 h, and the reaction was completed when the raw material was less than 0.5% by HPLC detection. The reaction mixture was cooled to 0-5°C, 378.39 g (3.00 mol) dimethyl sulfate was added dropwise within 2 h, and the reaction temperature was controlled at 0-5°C. After the dropwise addition was completed, the reaction was continued at this temperature for 2 h and then the temperature slowly rose to room temperature with much solid precipitated. After holding for 3 h, the conversion of intermediate **V-1** was basically completed, and the content of compound **VI-1** by HPLC detection was 90.3%. After adding 800 g of water and stirring for 30 min, white solid was obtained by filtration, and the filter cake was washed with water to give 437.07 g of wet product of the title compound **VI-1** with an HPLC area normalization purity of 97.7% (Method B). The wet product was directly used for subsequent reaction without additional treatment.

In a 3 L four-necked reaction flask, were placed 437.07 g of the above wet product of compound **VI-1** and 1200 g of acetic acid which were heated to 100°C, and 490 g concentrated sulfuric acid (98%) was slowly added dropwise with a dropwise addition time of 2 h. After the dropwise addition was completed, the reaction was continued for 8 h, and that the content of the raw material **VI-1** was less than 1.0% by HPLC detection was considered as the end of the reaction. After the reaction was completed, the temperature was cooled to 20-25°C, and 500 g of water was slowly added dropwise under the ice bath. After the dropwise addition was completed, the mixture was stirred at 10-15°C for 1 h and filtered. The filter cake was washed with water and dried to give 303.95 g of the title compound **I-2,** with a three-step yield of 82.9% and an HPLC area normalization purity of 98.3% (Method B).

### Example 15: Synthesis of compound I-2

In a 250 mL four-necked flask, were placed 60 g of DMAC and 20 g (76.4 mmol) of compound **IV-2** while stirring well. At room temperature, 13.76 g (76.4 mmol) of sodium methoxide-methanol solution (30%) was added dropwise, and after the dropwise addition, 15.38 g (84.0 mmol) of ethyl 3-amino-4,4,4-trifluorocrotonate was added. Then the mixture was heated to 90-100°C, and 15.12 g (84.0 mmol) of sodium methoxide-methanol solution (30%) was slowly added dropwise within 1 h while distilling and collecting the distilled methanol and ethanol. After the dropwise addition was completed, the reaction was continued at this temperature until there were no distilled methanol and ethanol. The reaction time was about 3 h, and the reaction was completed when the raw material was less than 0.5% by HPLC detection. The reaction mixture was cooled to 0-10°C, 42.68 g (229.2 mmol) methyl p-toluenesulfonate was added dropwise within 1 h, and the reaction temperature was controlled at 0-10°C. After the dropwise addition was completed, the reaction was continued at this temperature for 5 h and then the temperature slowly rose to room temperature and stayed overnight with much solid precipitated. The conversion of intermediate **V-1** was basically completed, and the content of compound **VI-1** by HPLC detection was 87.24% (Method B). After adding 60 g of water and stirring for 30 min, white solid was obtained by filtration, and the filter cake was washed with water to give 31.72 g of wet product of the title compound **VI-1.** The wet product was directly used for subsequent reaction without additional treatment.

In a 500 mL four-necked reaction flask, were placed 31.72 g of the above wet product of compound **VI-1** and 150 g of acetic acid which were heated to 100°C, and 30 g of concentrated sulfuric acid (98%) was slowly added dropwise with a dropwise addition time of 30 min. After the dropwise addition was completed, the reaction was continued for 3 h. The content of the remaining raw material **VI-1** was 1.6% by HPLC detection (Method B), and the raw material **VI-1** was reduced slowly when the reaction was continued. After the reaction was completed, the temperature was cooled to 20-25°C, and 50 g of water was slowly added dropwise under the ice bath. After the dropwise addition was completed, the mixture was stirred at 10-15°C for 1 h and filtered. The filter cake was washed with water and dried to give 21.99 g of the title compound **I-2,** with a three-step yield of 78.52% and an HPLC area normalization purity of 98.3% (Method B).

### Example 16: Synthesis of compound I-2

In a 250 mL four-necked flask, were placed 60 g of DMAC and 20 g (76.4 mmol) of compound **IV-2** while stirring well. At room temperature, 13.76 g (76.4 mmol) of sodium methoxide-methanol solution (30%) was added dropwise, and after the dropwise addition, 15.38 g (84.0 mmol) of ethyl 3-amino-4,4,4-trifluorocrotonate was added. Then the mixture was heated to 90-100°C, and 15.12 g (84.0 mmol) of sodium methoxide-methanol solution (30%) was slowly added dropwise within 1 h while distilling and collecting the distilled methanol and ethanol. After the dropwise addition was completed, the reaction was continued at this temperature until there were no distilled methanol and ethanol. The reaction time was about 3 h, and the reaction was completed when the raw material was less than 0.5% by HPLC detection. The reaction mixture was cooled to 0-10°C, 32.53 g (229.2 mmol) of methyl iodide was added dropwise within 1 h, and the reaction temperature was controlled at 0-10°C. After the dropwise addition was completed, the reaction was continued at this temperature for 5 h and the temperature slowly rose to room temperature and stayed overnight with much solid precipitated. The conversion of intermediate **V-1** was basically completed, and the content of compound **VI-1** by HPLC detection was 89.01% (Method B). After adding 60 g of water and stirring for 30 min, white solid was obtained by filtration, and the filter cake was washed with water to give 32.36 g of wet product of the title compound **VI-1.** The wet product was directly used for subsequent reaction without additional treatment.

In a 500 mL four-necked reaction flask, were placed 32.36 g of the above wet product of compound **VI-1** and 150.0 g of formic acid which were heated to 100°C, and 30.0 g concentrated sulfuric acid (98%) was slowly added dropwise with a dropwise addition of 30 min. After the dropwise addition was completed, the reaction was continued for 3 h. The content of the remaining raw of compound **VI-1** was 3.6% by HPLC detection (Method B), and the raw material **VI-1** was reduced slowly when the reaction was continued. After the reaction was completed, the temperature was cooled to 20-25°C, and 50 g of water was slowly added dropwise under the ice bath. After the dropwise addition was completed, the mixture was stirred at 10-15°C for 1 h and filtered. The filter cake was washed with water and dried to give 22.94 g of the title compound **I-2,** with a three-step yield of 81.89% and an HPLC area normalization purity of 97.1% (Method B).

### Example 17: Synthesis of compound I-2

In a 250 mL four-necked flask, were placed 60 g of DMAC and 20 g (76.4 mmol) of compound **IV-2** while stirring well. At room temperature, 13.76 g (76.4 mmol) of sodium methoxide-methanol solution (30%) was added dropwise, and after the dropwise addition, 15.38 g (84.0 mmol) of ethyl 3-amino-4,4,4-trifluorocrotonate was added. Then the mixture was heated to 90-100°C, and 15.12 g (84.0 mmol) of sodium methoxide-methanol solution (30%) was slowly added dropwise within 1 h while distilling and collecting the distilled methanol and ethanol. After the dropwise addition was completed, the reaction was continued at this temperature until there were no distilled methanol and ethanol. The reaction time was about 3 h, and the reaction was completed when the raw material was less than 0.5% by HPLC detection. The reaction mixture was cooled to 0-10°C, 37.61 g (0.2292 mol) methyl trifluoromethansulfonate was added dropwise for about 1 h, and much solid precipitated after the dropwise addition was completed. The conversion of intermediate **V-1** was basically completed, and the content of compound **VI-1** by HPLC detection was 88.12% (Method B). After adding 60 g of water and stirring for 30 min, white solid was obtained by filtration, and the filter cake was washed with water to give 32.06 g of wet product of the title compound **VI-1.** The wet product was directly used for subsequent reaction without additional treatment.

In a 500 mL four-necked reaction flask, were placed 32.06 g of the above wet product of compound **VI-1** and 150 g of formic acid which were heated to 100°C, and 60.0 g concentrated hydrochloric acid (36%) was slowly added dropwise with a dropwise addition time of 1 h. After the dropwise addition was completed, the reaction was continued for 8 h. The content of the remaining raw material **VI-1** was 2.12% by HPLC detection (Method B), and the raw material **VI-1** was reduced slowly when the reaction was continued. After the reaction was completed, the temperature was cooled to 20-25°C, and 100 g of water was slowly added dropwise under the ice bath. After the dropwise addition was completed, the mixture was stirred at 10-15°C for 1 h and filtered. The filter cake was washed with water and dried to give 22.22 g of the title compound **I-2,** with a three-step yield of 79.31% and an HPLC area normalization purity of 97.7% (Method B).

Similarly, compound **I-1** or **I-2** can be prepared by using other compounds of formula (**IV**) and ethyl 3-amino-4,4,4-trifluorocrotonate according to the methods in the above examples. Some of the examples were shown in Table 4:

**Table 4**

| Compound (**IV**) | Base (equiv.) | Solvent | Temperature (°C) | **I-1** Yield(%) | **I-1** Purity(%) |
|---|---|---|---|---|---|
| **IV-4** | sodium methoxide (2.4) | DMF | 90-100 | 92.31 | 98.30 |
| **IV-14** | sodium methoxide (2.5) | DMF | 90-100 | 90.31 | 92.42 |
| **IV-21** | sodium methoxide (2.4) | DMF | 90-100 | 92.45 | 96.14 |
| **IV-70** | sodium methoxide (2.4) | DMF | 90-100 | 89.74 | 98.56 |

### Example 18: Synthesis of compound III-5

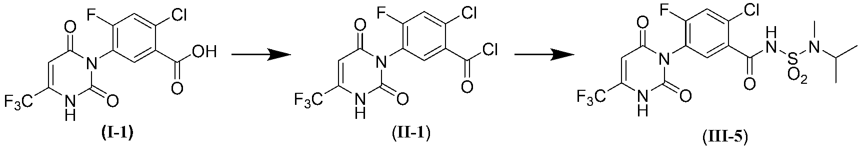

In a 1 L four-necked flask, were placed 500 g of 1,2-DCE, 105.79 g (0.30 mol) compound **I-1** and 1.83 g (0.015 mol) of DMAP. The mixture was heated to 80°C, and 42.84 g (0.36 mol) of thionyl chloride was slowly added dropwise with a dropwise addition time of 2 h. After the dropwise addition was completed, the reaction was performed at this temperature for 3 h to give a solution of compound **II-1.** The solution was heated to about 83°C, and the reflux state was maintained. 20-30 g of fraction was distilled at atmospheric pressure, which was a mixture of the remaining thionyl chloride and 1,2-DCE. The residue was cooled to room temperature for use. HPLC qualitative content of compound **II-1** was 97.5% (methanol derivative). ¹H NMR (400 MHz, CD₃CN) δ 9.90 (s, 1H), 8.30 (d, *J =* 7.5 Hz, 1H), 7.59 (d, *J =* 9.3 Hz, 1H), 6.26 (s, 1H).

In a 1 L four-necked flask, were placed 54.80 g (0.36 mol) of N-methyl-N-isopropylsulfamoyl amide, 91.08 g (0.90 mol) of triethylamine, 80 g of 1,2-DCE and 7.33 g (0.06 mol) of DMAP while stirring, which were heated to 60°C. The above 1,2-DCE solution of the compound **II-1** was added dropwise into the four-necked flask with a dropwise addition time of 2 h. After the dropwise addition was completed, the reaction was performed at this temperature for 1 h, and the reaction was completed when the content of raw material was less than 0.2 % by HPLC detection. After adding 400 g of water and stirring for 10 min, 60.83 g (0.6 mol) of 36% hydrochloric acid was slowly added dropwise with a large amount of white solid precipitated. 192.64 g of wet product of compound **III-5** was obtained by filtration, with an HPLC area normalization purity of98.22 % (Method B). ¹H NMR (400 MHz, DMSO-d6) δ 12.83 (s, 1H), 12.27 (s, 1H), 7.81 (dd, *J =* 19.1, 8.3 Hz, 2H), 6.46 (s, 1H), 4.32 - 3.92 (m, 1H), 2.83 (s, 3H), 1.14 (d, *J =* 6.2 Hz, 6H). mp 242.9-244.7°C (decomposition).

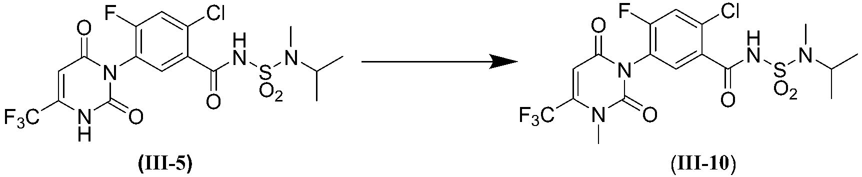

In a 1 L four-necked flask, was placed a mixed solvent of toluene, tetrahydrofuran, and water while stirring well. Then, the wet product of compound **III-5** (192.64 g, 0.28 mol), tetrabutylammonium bromide (9.35 g, 0.029 mol) and dimethyl sulfate (64.50 g, 0.51 mol) were sequentially added to the mixed solvent at 25°C, and the mixture was heated to 40-45°C. The pH value of the reaction mixture was controlled between 4-5 by adding 10% NaOH aqueous solution dropwise at 40-45°C, and the reaction was completed until the raw material was less than 1.5% (HPLC, Method B). After extraction and separation, the organic phase was washed with water and the solvent was removed by reduced pressure distillation. The residue was recrystallized, and the filter cake was washed with a small amount of toluene after filtration. The obtained wet product was dried to give 106.22 g of white solid of compound **III-10,** with a three-step yield of 70.69 % and an HPLC area normalization purity of 97.5 % (Method B). ¹H NMR (400 MHz, DMSO) δ 12.28 (s, 1H), 7.85 (d, J = 9.6 Hz, 1H), 7.72 (d, J = 7.6 Hz, 1H), 6.61 (s, 1H), 4.17 - 4.04 (m, 1H), 3.42 (s, 3H), 2.82 (s, 3H), 1.14 (d, J = 6.7 Hz, 6H). mp 186.8-190.3°C.

WO2005054208A1 specifically disclosed compound **III-5** and compound **III-10** (Saflufenacil).

2-chloro-5-[3,6-dihydro-2,6-dioxo-4-(trifluoromethyl)-1(2H)-pyrimidinyl]-4-fluoro-N-[[methyl (1-methylethyl)amino]sulfonyl]benzamide:
mp 233-236°C (decomposition).¹H-NMR (400 MHz, DMSO-*d6*) δ (ppm): 12,8 (br, NH), 12,25 (s, NH), 7,82 (d, 1H), 7,76 (d, 1H), 6,4 (s, 1H), 4,1 (m, 1H), 2,8 (s, 3H), 1,12 (d, 3H), 1,12 (d, 6H).

2-chloro-5-[3,6-dihydro-3-methyl-2,6-dioxo-4-(trifluoromethyl)-1(2H)-pyrimidinyl]-4-fluoro-N-[[methyl(1-methylethyl)amino]sulfonyl]benzamide(Saflufenacil):
¹H-NMR(400 MHz, CDCl₃)δ(ppm): 9.5(br, NH), 7.63(d, 1H), 7.37(d, 1H), 6.37(s, 1H), 4.29(m, 1H), 3.58(s, 3H), 2.92(s, 3H), 1.18(d, 6H).

### Example 19: Synthesis of compound III-7

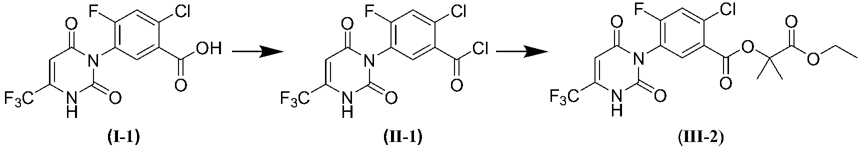

In a 1 L four-necked flask, were placed 500 g of 1,2-DCE, 105.79 g (0.30 mol) compound **I-1** and 1.83 g (0.015 mol) DMAP. The mixture was heated to 80°C, and 45.69 g (0.36 mol) of oxalyl chloride was slowly added dropwise with a dropwise addition time of 2 h. After the dropwise addition was completed, the reaction was performed at this temperature for 3 h to give a solution of compound **II-1.** The solution was heated to about 83°C, and the reflux state was maintained. 20-30 g of fraction was distilled at atmospheric pressure, which was a mixture of the remaining oxalyl chloride and 1,2-DCE. The residue was cooled to room temperature for use. HPLC qualitative content of compound **II-1** was 97.2% (Method B, methanol derivative).

In a 1 L four-necked flask, were placed 47.58 g (0.36 mol) of ethyl 2-hydroxyisobutyrate, 91.08 g (0.90 mol) of triethylamine, 80 g of 1,2-DCE and 7.33 g (0.06 mol) of DMAP while stirring, which were heated to 60°C. The 1,2-DCE solution containing the compound **II-1** (0.30 mol) was added dropwise into the four-necked flask with a dropwise addition time of 2 h. After the dropwise addition was completed, the reaction was performed at this temperature for 1 h, and the reaction was completed when the content of raw material was less than 0.2 % by HPLC detection. The reaction mixture was added with 400g of water and stirred for 10 min, standing for layering. The organic phase was separated and distilled under reduced pressure to remove the solvent to give 132.22 g of light yellow oil of compound **III-2** with a yield of 94.42% and an HPLC area normalization purity of 93.8% (Method B). ¹H NMR (400 MHz, CDCl₃) δ 10.52 (s, 1H), 7.87 (d, *J* = 7.7 Hz, 1H), 7.40 (s, 1H), 6.25 (s, 1H), 4.23 (q, *J =* 7.1 Hz, 2H), 1.68 (d, *J =* 2.5 Hz, 6H), 1.26 (t, *J =* 7.1 Hz, 3H).

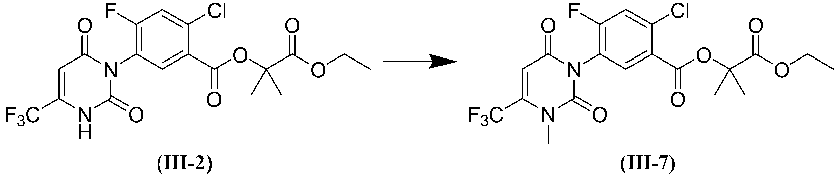

In a reaction flask, were placed 93.55 g (0.2 mol) of compound **III-2,** 55.28 g (0.4 mol) of potassium carbonate and 400 g of N,N-dimethylformamide while stirring for 0.5 h at room temperature, and 30.27 g (0.24 mol) dimethyl sulfate was added dropwise slowly. After the dropwise addition was completed, the reaction was continued until the raw material was disappeared by detection. The reaction mixture was filtered, and the mother liquid was distilled under reduced pressure to remove part of DMF, poured into 500 g of water, and extracted with 1000 g of ethyl acetate. The organic phase was washed twice with saturated salt water and spin-dried to give 83.39 g of the title compound **III-7** with a yield of 86.72 % and an HPLC area normalization purity of 92.1% (Method B). 85 g of isopropanol aqueous solution (80%) was added for recrystallization, and powdered white solid was obtained by low temperature filtration and dried to give 78.11 g of compound **III-7** with a yield of 81.23 % and an HPLC area normalization purity of 97.7 %. ¹H NMR (400 MHz, DMSO) δ 8.07 (d, *J =* 7.8 Hz, 1H), 7.91 (d, *J =* 9.6 Hz, 1H), 6.62 (s, 1H), 4.16 (q, *J* = 7.1 Hz, 2H), 3.43 (s, 3H), 1.62 (s, 6H), 1.19 (t, *J* = 7.1 Hz, 3H).

### Example 20: Synthesis of compound III-8

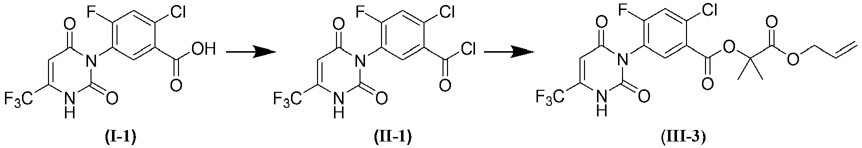

In a 1 L four-necked flask, were placed 500 g of 1,2-DCE, 105.79 g (0.30 mol) compound **I-1** and 1.83 g (0.015 mol) DMAP. The mixture was heated to 80°C, and 42.84 g (0.36 mol) of thionyl chloride was slowly added dropwise with a dropwise addition time of 2 h. After the dropwise addition was completed, the reaction was performed at this temperature for 3 h to give a solution of compound **II-1.** The solution was heated to about 83°C, and the reflux state was maintained. 20-30 g of fraction was distilled at atmospheric pressure, which was a mixture of the remaining thionyl chloride and 1,2-DCE. The residue was cooled to room temperature for use. HPLC qualitative content of compound **II-1** was 97.5% (methanol derivative).

In a 250 mL four-necked flask, were placed 17.30 g (0.12 mol) of allyl 2-hydroxy-2-methylpropionate (prepared according to the method recited in WO2009046665), 30.36 g (0.30 mol) of triethylamine, 20 g of 1,2-DCE and 2.44 g (0.02 mol) of DMAP while stirring, which were heated to 60°C. The 1,2-DCE solution containing the compound **II-1** (0.10 mol) was added dropwise into the four-necked flask with a dropping time of 1 h. After the dropwise addition was completed, the reaction was performed at this temperature for 1 h, and the reaction was completed when the content of raw material was less than 0.2 % by HPLC detection. The reaction mixture was added with 100 g of water and stirred for 10 min, standing for layering. The organic phase was separated and distilled under reduced pressure to remove the solvent to give 42.75 g of light yellow oil of compound **III-3** with a yield of 89.29% and an HPLC area normalization purity of 92.3%. ¹H NMR (400 MHz, CDCl₃) δ 10.49 (s, 1H), 7.79 (d, *J* = 7.7 Hz, 1H), 7.30 (d, *J* = 9.1 Hz, 1H), 6.17 (s, 1H), 5.82 (ddt, *J =* 16.2, 10.7, 5.7 Hz, 1H), 5.19 (ddd, *J =* 13.8, 11.5, 1.2 Hz, 2H), 4.58 (d, *J =* 5.7 Hz, 2H), 1.62 (d, *J* = 2.3 Hz, 6H).

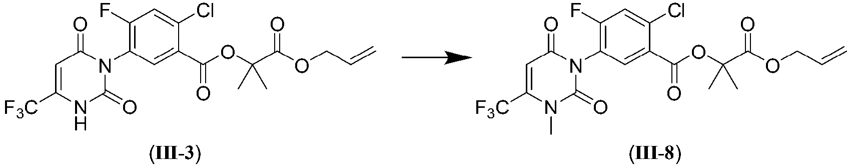

In a reaction flask, were placed 23.94 g (0.05 mol) of compound **III-3,** 13.82 g (0.1 mol) of potassium carbonate and 90 g of N,N-dimethylformamide while stirring for 0.5 h at room temperature, and 7.57 g (0.06 mol) of dimethyl sulfate was slowly added dropwise. After the dropwise addition was completed, the reaction was continued until the raw material was disappeared by detection. The reaction mixture was filtered, and the mother liquid was distilled under reduced pressure to remove part of DMF, poured into 100 g of water, and extracted with 200 g of ethyl acetate. The organic phase was washed twice with saturated salt water and spin-dried to give 20.63 g of oil of the title compound **III-8** with a yield of 83.71 % and an HPLC area normalization purity of 91.7%. ¹H NMR (400 MHz, DMSO) δ 8.09 (d, *J =* 7.8 Hz, 1H), 7.91 (d, *J =* 9.6 Hz, 1H), 6.62 (s, 1H), 5.90 (ddt, *J =* 17.2, 10.7, 5.4 Hz, 1H), 5.37 - 5.29 (m, 1H), 5.22 (ddd, *J =* 10.5, 2.7, 1.3 Hz, 1H), 4.64 (dt, *J =* 5.4, 1.4 Hz, 2H), 3.43 (s, 3H), 1.65 (s, 6H).

### Example 21: Synthesis of compound III-9

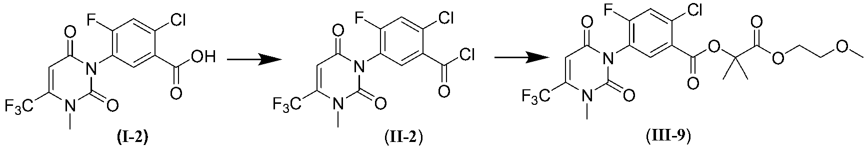

In a 1 L four-necked flask, were placed 550 g of 1,2-DCE, 110.00 g (0.30 mol) compound **I-2** and 1.83 g (0.015 mol) DMAP. The mixture was heated to 80°C, and 42.84 g (0.36 mol) of thionyl chloride was slowly added dropwise with a dropwise addition time of 2 h. After the dropwise addition was completed, the reaction was performed at this temperature for 3 h to give a solution of compound **II-2.** The solution was heated to about 83°C, and the reflux state was maintained. 20-30 g of fraction was distilled at atmospheric pressure, which was a mixture of the remaining thionyl chloride and 1,2-DCE. The residue was cooled to room temperature for use. HPLC qualitative content of compound **II-2** was 97.4% (methanol derivative). ¹H NMR (400 MHz, CD₃CN) δ 8.27 (d, J = 7.5 Hz, 1H), 7.59 (d, J = 9.3 Hz, 1H), 6.38 (s, 1H), 3.44 (d, J = 1.2 Hz, 3H).

In a 1 L four-necked flask, were placed 58.38 g (0.36 mol) of 2-methoxyethyl 2-hydroxy-2-methylpropionate (prepared according to the literature method), 45.54 g (0.45 mol) of triethylamine, 80 g of 1,2-DCE and 1.83 g (0.015 mol) of DMAP while stirring, which were heated to 60°C. The 1,2-DCE solution containing the compound **II-2** (0.3 mol) was added dropwise into the four-necked flask with a dropwise addition time of 2 h. After the dropwise addition was completed, the reaction was performed at this temperature for 1 h, and the reaction was completed when the content of raw material was less than 0.2 % by HPLC detection. The reaction mixture was added with 400g of water and stirred for 10 min, standing for layering. The organic phase was separated and distilled under reduced pressure to remove the solvent to give 145.40 g of light yellow oil of compound **III-9** with a yield of 95.1% and an HPLC area normalization purity of 96.8% (Method B).120 g of isopropanol was added for recrystallization, and powdered white solid was obtained by filtration and dried to give 129.95 g of compound **III-9** with a yield of 84.8% and an HPLC area normalization purity of 99.2% (Method B). ¹H NMR (400 MHz, DMSO-*d*₆) δ 8.07 (d, *J =* 7.8 Hz, 1H), 7.92 (d, *J* = 9.6 Hz, 1H), 6.63 (s, 1H), 4.25 - 4.17 (m, 2H), 3.52 - 3.49 (m, 2H), 3.42 (d, *J =* 1.3 Hz, 3H), 2.51 (d, *J=* 1.8 Hz, 3H), 1.63 (s, 6H).

The reference information of the preparation method of 2-methoxyethyl 2-hydroxy-2-methylpropionate was as follows: Preparation of allyl and methallyl methacrylates by the thermal decomposition of allyl and methallyl α-acetoxyisobutyrates, By: Rehberg, Chessie E.; et al, Journal of the American Chemical Society (1943), 65, 1003-6.

### Example 22: Synthesis of compound II-2

In a 1 L four-necked flask, were placed 500 g toluene, 110.00 g (0.30 mol) compound **I-2** and 1.83 g (0.015 mol) of DMAP. The mixture was heated to 60°C, and the toluene solution containing 44.51 g (0.15 mol) of triphosgene was slowly added dropwise with a dropwise addition time of 2 h. After the dropwise addition was completed, the reaction was performed at this temperature for 3 h to give a solution of compound **II-2.** The solution was heated to about 110°C, and the reflux state was maintained while stirring for 2 h. The overflowing exhaust gas was collected and absorbed with sodium hydroxide aqueous solution, and the reaction mixture was cooled to room temperature for use. HPLC qualitative content of compound **II-2** was 97.8 % (methanol derivative).

### Example 23: Synthesis of compound II-2

In a 1 L four-necked flask, were placed 550 g of 1,2-DCE, 110.00 g (0.30 mol) of compound **I-2** and 1.83 g (0.015 mol) of DMAP. The mixture was heated to 80°C, and 45.69 g (0.36 mol) of oxalyl chloride was slowly added dropwise with a dropwise addition time of 2 h. After the dropwise addition was completed, the reaction was performed at this temperature for 3 h to give a solution of compound **II-2.** The solution was heated to about 83°C, and the reflux state was maintained. 20-30 g of fraction was distilled at atmospheric pressure, which was a mixture of the remaining oxalyl chloride and 1,2-DCE. The residue was cooled to room temperature for use. HPLC qualitative content of compound **II-2** was 97.5% (methanol derivative).

### Example 24: Synthesis of compound II-2

In a 100 mL four-necked flask, were placed 55 g of chlorobenzene, 11.00 g (0.03 mol) of compound **I-2** and 0.13 g (0.0015 mol) of DMF. The mixture was heated to 80°C, and a chlorobenzene solution containing 3.56 g (0.012 mol) of triphosgene was slowly added dropwise with a dropwise addition time of 2 h. After the dropwise addition was completed, the reaction was performed at this temperature for 2 h to give a solution of compound **II-2.** The solution was heated to about 110°C, the reflux state was maintained while stirring for 2 h, and the reaction mixture was cooled to room temperature for use. HPLC qualitative content of compound **II-2** was 98.27% (methanol derivative).

### Example 25: Synthesis of compound II-2

In a 100 mL four-necked flask, were placed 55 g of xylene, 11.00 g (0.03 mol) of compound **I-2** and 0.130 g (0.0015 mol) of DMF. The mixture was heated to 80°C, and a xylene solution containing 3.56 g (0.012 mol) of triphosgene was slowly added dropwise with a dropwise addition time of 2 h. After the dropwise addition was completed, the reaction was performed at this temperature for 2 h to give a solution of compound **II-2.** The solution was heated to about 110°C, the reflux state was maintained while stirring for 2 h, and the reaction mixture was cooled to room temperature for use. HPLC qualitative content of compound **II-2** was 96.52% (methanol derivative).

### Biological example 1

### Determination of antifungal activity

Complete medium (CM): 20×nitrate salts 50 mL, trace elements 1 mL, vitamin solution 1 mL, D-Glucose 10 g, peptone 2 g, yeast extract 1 g, casamino acids 1 g, and dd H₂O (double distilled water) was added to a constant volume of 1 L. The preparation of solid medium required the addition of 1.5% agar powder after packaging, high-pressure sterilization at 121°C for 20 min, and storage at room temperature. 20×nitrate salts: NaNO₃ 120 g, KCl 10.4 g, MgSO₄·7H₂O 10.4 g, KH₂PO₄ 30.4 g, and dd H₂O (double distilled water) was added to a constant volume of 1 L, which was performed high-pressure sterilization at 121°C for 20 min and stored at 4°C.

Trace elements: ZnSO₄·7H₂O 2.2 g, H₃BO₃ 1.1 g, MnCl₂·4H₂O 0.5 g, FeSO₄·7H₂O 0.5 g, CoCl₂·6H₂O 0.17 g, CuSO₄·5H₂O 0.16 g, Na₂MoO₄·2H₂O 0.15 g, and dd H₂O (double distilled water) was added to a constant volume of 100 mL, which was stored at 4°C.

Vitamin solution: biotin 0.1 g, thiamine 0.1 g, riboflavin 0.1 g, pyridoxin 0.1 g, nicotinic acid 0.1 g, para aminobenzoic acid 0.1 g, and dd H₂O (double distilled water) was added to a constant volume of 100 mL, which was stored away from light at 4°C.

PSA medium: the potato was washed and peeled, 200 g of which was cut into small pieces, boiled with 1 L ddH₂O (double distilled water) for 30 min, filtered with two layers of gauze to discard the residue, added with 20 g of sucrose while stirring evenly, cooled slightly, and added with ddH2O (double distilled water) to a constant volume of 1 L. 1.5 % agar powder was added after packaging, which was performed high-pressure sterilization at 121°C for 20 min and stored at room temperature.

PDA medium (potato agar glucose medium): 200 g of potatoes were washed and peeled, cut into small pieces, boiled in boiling water (boiled for 30 min), filtered with four layers of gauze to removed the filter residue, added with 20 g of glucose and 20 g of agar, and added with dd H₂O (double distilled water) to a constant volume of 1000 mL while stirring to make it fully dissolved, which was divided into 250 mL conical flasks, sterilized at 121°C for 20 min, and cooled for later use.

Rye medium: two portions of 30 g of rye were weighed and placed in a 1 L conical flask respectively, and 500 mL ultrapure water was added to each flask. Sterilization was performed at 121°C for 20 min to obtain softened rye, which was crushed to homogenate by a juicer, filtered to obtain rye juice, and diluted to 1 L. 2.7 g of agar and 4 g of sucrose was weighed and placed in a 250 mL conical flask, and 200 mL rye juice was added to each flask, which was sterilized at 121°C for 20 min and cooled for later use.

### Determination objects: Fusarium graminearum, Fusarium asiaticum, Magnaporthe oryzae, Ustilaginoidea virens, Phytophthora infestans.

Determination method: The compound of formula (**IV**) was dissolved in DMSO, which was added to the corresponding medium. The determination concentration was set at 1500 µg/mL to make a drug-containing plate. Blank control was set and repeated for 3 times. The plate method was used. The cultured phytopathogenic fungi were cut from the edge of the colony to obtain a mycelial plug using a sterilizing puncher with a diameter of 5 mm under sterile conditions, and the mycelial plug was inoculated into the center of the drug-containing plate with an inoculator. The mycelium was facing up and covered with a cover. When the colony of the control group was full, the diameter of the colony was measured, and the percentage of inhibition of mycelial growth by each drug treatment was calculated. Among them, *F*. *graminearum* and *F. asiaticum* were cultured in PDA medium in an incubator away from light at 25°C; *M. oryzae* was cultured in CM medium in an incubator away from light at 28°C; *U. virens* was cultured in PSA medium in an incubator away from light at 25°C; and *P. infestans* was cultured in rye medium in an incubator away from light at 18°C.

Mycelial growth inhibition rate (%) = (colony diameter of blank control - colony diameter of drug treatment) / (colony diameter of blank control - 5) * 100.

The test results show that:
the following compounds show an inhibition rate of 100% against *M. oryzae* at a concentration of 1500 µg/mL: **IV-5, IV-9, IV-14, IV-69, IV-71;**
the following compounds show an inhibition rate of 100% against *U. virens* at a concentration of 1500 µg/mL: **IV-9, IV-14, IV-21;**
the following compounds show an inhibition rate of 100% against *P*. *infestans* at a concentration of 1500 µg/mL: **IV-1, IV-2, IV-3, IV-4, IV-5, IV-6, IV-9, IV-14, IV-21, IV-33, IV-38, IV-69, IV-70, IV-71.**

The above-mentioned examples are only preferred examples of the present invention. It should be pointed out that for the person skilled in the art, some changes and improvements can be made without deviating from the idea of the present invention, which should be within the protection scope of the present invention.

## Claims

1. A method for preparing a compound of formula (**III**) with the following reaction scheme, comprising: 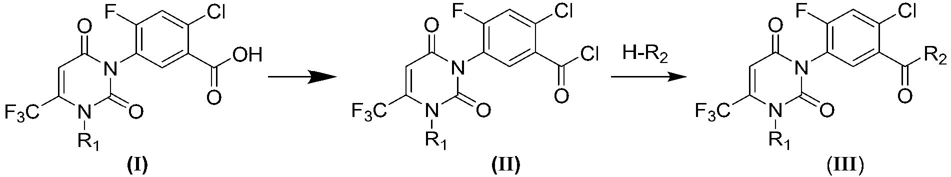
reacting a compound of formula (**I**) with a chlorinating reagent in the presence of a base A in an organic solvent A at 20°C-120°C to give a compound of formula (**II**)**;** wherein, the organic solvent A is selected from at least one of dichloromethane, chloroform, 1,2-dichloroethane, chlorobenzene, dichlorobenzene, toluene or xylene; the base A is selected from at least one of N,N-dimethylformamide, 4-pyrrolidinopyridine or 4-dimethylaminopyridine; the amount of base A is 0.001-0.2 equivalent of the compound of formula (**I**); the chlorinating reagent is selected from SOCl₂, POCl₃, PCl₅, ClCOCOCl, COCl₂, ClCOOCCl₃ or Cl₃COCOOCCl₃;
reacting a compound of formula (**II**) with H-R₂ in the presence of a base B in an organic solvent B at 0°C-120°C to give a compound of formula (**III**); wherein, the organic solvent B is selected from at least one of dichloromethane, chloroform, 1,2-dichloroethane, chlorobenzene, dichlorobenzene, toluene, xylene, tetrahydrofuran or 2-methyltetrahydrofuran; the base B is selected from at least one of 4-dimethylaminopyridine, trimethylamine, triethylamine, diisopropylethylamine, pyridine, 2-methylpyridine, 3-methylpyridine, 1,8-diazabicyclo[5.4.0]undec-7-ene(DBU) or 2,6-lutidine; the amount of base B is 1.0-3.0 equivalents of the compound of formula (**II**)**;**
R₁ is selected from H or -CH₃;
R₂ is selected from -OC(CH₃)₂COOCH₃, -OC(CH₃)₂COOCH₂CH₃, - OC(CH₃)₂COOCH₂CH₂OCH₃, -OC(CH₃)₂COOCH₂CH=CH₂ or -NHSO₂N(CH₃)*i*-Pr.

2. The method according to claim 1, wherein
R₁ is selected from H;
R₂ is selected from -OC(CH₃)₂COOCH₃, -OC(CH₃)₂COOCH₂CH₃, - OC(CH₃)₂COOCH₂CH₂OCH₃, -OC(CH₃)₂COOCH₂CH=CH₂ or -NHSO₂N(CH₃)*i*-Pr.

3. The method according to claim 1, wherein
R₁ is selected from -CH₃;
R₂ is selected from -OC(CH₃)₂COOCH₃, -OC(CH₃)₂COOCH₂CH₃, - OC(CH₃)₂COOCH₂CH₂OCH₃, -OC(CH₃)₂COOCH₂CH=CH₂ or -NHSO₂N(CH₃)*i*-Pr.

4. A compound of formula (**II**): 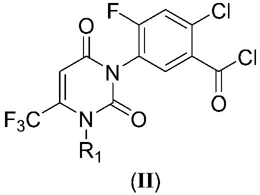 wherein R₁ is selected from H.

5. Use of the compound of formula (**II**) according to claim 4 in the preparation of the compound of formula (**III**) according to claim 2.

6. A method for preparing a compound of formula (**I**) with the following reaction scheme, comprising: 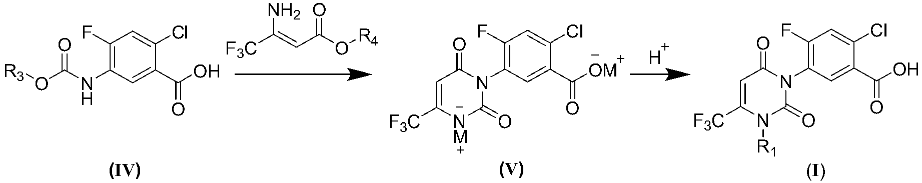
reacting a compound of formula (**IV**) with an R₄ ester of 3-amino-4,4,4-trifluorocrotonic acid in the presence of a base C in an organic solvent C at -20°C to the boiling point of the solvent to give a compound of formula (**V**);
treating the compound of formula (**V**) with an inorganic acid to give the compound of formula (**I**);
wherein,
the base C is selected from at least one of CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the organic solvent C is selected from at least one of methanol, ethanol, isopropanol, tert-butanol, acetonitrile, tetrahydrofuran, 2-methyltetrahydrofuran, 1,4-dioxane, toluene, o-xylene, m-xylene, p-xylene, xylene, 4-methyl-2-pentanone, cyclohexanone, N-methylpyrrolidone, N,N-dimethylformamide, N,N-dimethylacetamide, or dimethyl sulfoxide;
R₁ is selected from H;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl;
R₄ is selected from C₁-C₄ alkyl;
M is selected from K, Na, Li or Cs.

7. The method according to claim 6, wherein
R₃ is selected from C₁-C₇ alkyl, C₃-C₆ cycloalkyl, C₃-C₆ alkenyl, C₃-C₆ alkynyl, phenyl or benzyl;
R₄ is selected from methyl or ethyl;
M is selected from K, or Na;
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the amount of base C is 1.0-3.0 equivalents of the compound of formula (**IV**);
the inorganic acid is selected from hydrochloric acid, sulfuric acid, nitric acid or hydrobromic acid;
the organic solvent C is selected from at least one of methanol, ethanol, tert-butanol, acetonitrile, N-methylpyrrolidone, N,N-dimethylformamide, N,N-dimethylacetamide or dimethyl sulfoxide;
the reaction temperature is selected from 0°C-120°C.

8. The method according to claim 7, wherein
R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl;
R₄ is selected from methyl or ethyl;
M is selected from K, or Na;
the base C is selected from CH₃OK, CH₃CH₂OK, (CH₃)₃COK, CH₃ONa, CH₃CH₂ONa, or (CH₃)₃CONa;
the amount of the base C is 1.0-3.0 equivalents of the compound of formula (**IV**);
the inorganic acid is selected from hydrochloric acid;
the organic solvent C is selected from at least one of methanol, ethanol, acetonitrile, N-methylpyrrolidone, N,N-dimethylformamide, or N,N-dimethylacetamide;
the reaction temperature is selected from 0°C-100°C.

9. A method for preparing a compound of formula (**I**) with the following reaction scheme, comprising: 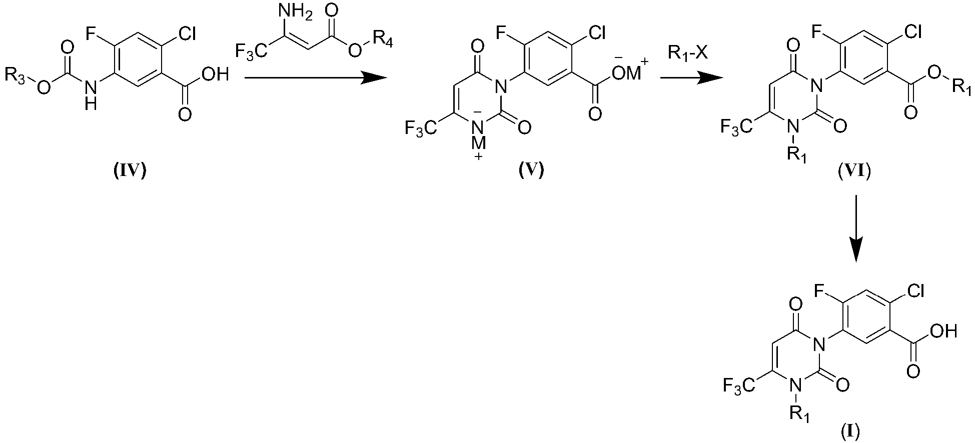
reacting a compound of formula (**IV**) with an R₄ ester of 3-amino-4,4,4-trifluorocrotonic acid in the presence of a base C in an organic solvent C at -20°C to the boiling point of the solvent to give a compound of formula (**V**);
reacting the compound of formula (**V**) with R₁-X in an organic solvent C at -20°C to the boiling point of the solvent to give a compound of formula (**VI**);
reacting the compound of formula (**VI**) with an inorganic acid in an organic solvent D at 0°C to the boiling point of the solvent to give the compound of formula (**I**);
wherein,
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the organic solvent C is selected from at least one of methanol, ethanol, isopropanol, tert-butanol, acetonitrile, tetrahydrofuran, 2-methyltetrahydrofuran, 1,4-dioxane, toluene, o-xylene, m-xylene, p-xylene, xylene, 4-methyl-2-pentanone, cyclohexanone, N-methylpyrrolidone, N,N-dimethylformamide, N,N-dimethylacetamide, or dimethyl sulfoxide;
the organic solvent D is selected from formic acid or acetic acid;
R₁ is selected from -CH₃;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl;
R₄ is selected from C₁-C₄ alkyl;
M is selected from K, Na, Li or Cs;
X is selected from halogen, -OSO₂OCH₃, -OMs, -OTs or -OTf.

10. The method according to claim 9, wherein
R₃ is selected from C₁-C₇ alkyl, C₃-C₆ cycloalkyl, C₃-C₆ alkenyl, C₃-C₆ alkynyl, phenyl or benzyl;
R₄ is selected from methyl or ethyl;
M is selected from K, or Na;
X is selected from Cl, Br, I, -OSO₂OCH₃ or -OMs;
the base C is selected from CH₃OK, CH₃CH₂OK, (CH₃)₃COK, CH₃ONa, CH₃CH₂ONa, (CH₃)₃CONa, K₂CO₃, Na₂CO₃, Cs₂CO₃, KOH, NaOH, LiOH, NaH or NaNH₂;
the amount of the base C is 1.0-3.0 equivalents of the compound of formula (**IV**);
the inorganic acid is selected from hydrochloric acid, sulfuric acid, or nitric acid;
the organic solvent C is selected from at least one of methanol, ethanol, tert-butanol, N-methylpyrrolidone, N,N-dimethylformamide, N,N-dimethylacetamide, or dimethyl sulfoxide;
the organic solvent D is selected from acetic acid;
the reaction temperature is selected from 0-120°C.

11. The method according to claim 10, wherein
R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl;
R₄ is selected from methyl or ethyl;
M is selected from K, or Na;
X is selected from I, or -OSO₂OCH₃;
the base C is selected from CH₃OK, CH₃CH₂OK, (CH₃)₃COK, CH₃ONa, CH₃CH₂ONa, or (CH₃)₃CONa;
the amount of the base C is 1.0-3.0 equivalents of the compound of formula (**IV**);
the inorganic acid is selected from hydrochloric acid, or sulfuric acid;
the organic solvent C is selected from methanol, ethanol, N-methylpyrrolidone, N,N-dimethylformamide, or N,N-dimethylacetamide;
the organic solvent D is selected from acetic acid;
the reaction temperature is selected from 0-110°C.

12. A method for preparing a compound of formula (**IV**) with the following reaction scheme, comprising: 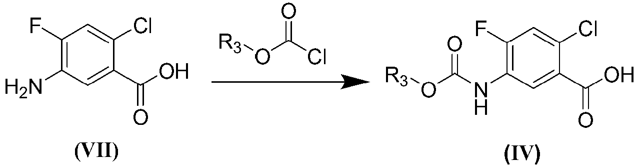
reacting a compound of formula (**VII**) with a chloroformate of formula R₃OCOCl in an organic solvent E at -20°C to the boiling point of the solvent to give a compound of formula (**IV**);
wherein,
the organic solvent E is selected from at least one of dichloromethane, chloroform, 1,2-dichloroethane, toluene, xylene, chlorobenzene, dichlorobenzene, N-methylpyrrolidone, ethylene glycol dimethyl ether, ethylene glycol diethyl ether, diethylene glycol diethyl ether, 1,4-dioxane, tetrahydrofuran, or 2-methyltetrahydrofuran;
R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl.

13. The method according to claim 12, wherein
R₃ is selected from C₁-C₇ alkyl, C₃-C₆ cycloalkyl, C₃-C₆ alkenyl, C₃-C₆ alkynyl, phenyl or benzyl;
the organic solvent E is selected from toluene, ethylene glycol dimethyl ether, 1,4-dioxane, tetrahydrofuran, 2-methyltetrahydrofuran, or N-methylpyrrolidone;
the reaction temperature is selected from 60-110°C.

14. The method according to claim 13, wherein
R₃ is selected from methyl, ethyl, n-propyl, isopropyl, n-butyl, tert-butyl, isobutyl, n-pentyl, n-hexyl, n-heptyl, cyclopentyl, allyl, phenyl or benzyl;
the organic solvent E is selected from ethylene glycol dimethyl ether, 1,4-dioxane, tetrahydrofuran, 2-methyltetrahydrofuran, or N-methylpyrrolidone.

15. A compound of formula (**IV**): 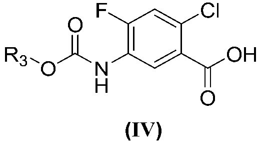 wherein R₃ is selected from C₁-C₁₀ alkyl, C₃-C₁₂ cycloalkyl, C₃-C₆ cycloalkyl C₁-C₆ alkyl, C₃-C₆ alkenyl, C₃-C₆ alkynyl, C₁-C₈ halogenated alkyl, C₁-C₆ alkoxy C₁-C₆ alkyl, C₃-C₆ oxygen-containing saturated heterocyclyl, C₃-C₆ oxygen-containing saturated heterocyclyl C₁-C₆ alkyl, phenyl or benzyl.

16. The compound of formula (**IV**) according to claim 15, wherein R₃ is selected from C₁-C₇ alkyl, C₃-C₆ cycloalkyl, C₃-C₆ alkenyl, C₃-C₆ alkynyl, phenyl or benzyl.

17. The compound of formula **(IV)** according to claim 16, wherein R₃ is selected from methyl, ethyl, n-propyl, isopropyl, n-butyl, isobutyl, tert-butyl, n-pentyl, n-hexyl, n-heptyl, cyclopentyl, allyl, phenyl or benzyl.

18. Use of the compound of formula **(IV)** according to anyone of claims 15-17 in the prevention and control of plant diseases.

19. A fungicidal composition, comprising, as an active substance, a compound of formula (IV) according to any one of claims 15-17 or an agriculturally acceptable salt thereof, and an agriculturally acceptable carrier, wherein the active substance is present in an amount of 1-99 wt%.

20. Use of the compound of formula **(IV)** according to anyone of claims 15-17 in the preparation of the compound of formula **(I)** according to anyone of claims 1, 6 and 9.
